# EUROPÄISCHE PATENTSCHRIFT

(11) **EP 3 131 907 B1**
(45) Veröffentlichungstag und Bekanntmachung des Hinweises auf die Patenterteilung: **28.02.2018**
(21) Anmeldenummer: 15720285.4
(22) Anmeldetag: 16.04.2015
(51) Int. Cl.: C07F 7/00, C08G 77/26

(54) **AMIDIN - ODER GUANIDINGRUPPEN-HALTIGES SILAN**
AMIDINE GROUP- OR GUANIDINE GROUP-CONTAINING SILANE
SILANE CONTENANT DES GROUPES AMIDINE OU GUANIDINE

(30) Priorität: 16.04.2014 EP 14164920
(43) Veröffentlichungstag der Anmeldung: 22.02.2017
(73) Patentinhaber: Sika Technology AG, 6340 Baar (CH)
(72) Erfinder: BURCKHARDT, Urs, CH-8049 Zürich (CH); CANNAS, Rita, CH-8600 Dübendorf (CH)
(74) Vertreter: Sika Patent Attorneys
(86) Internationale Anmeldenummer: PCT/EP2015/058333
(87) Internationale Veröffentlichungsnummer: WO 2015/158864

(56) Entgegenhaltungen:
- FR-A1- 2 925 496
- JP-A- 2002 167 438

## Beschreibung

### Technisches Gebiet

Die Erfindung betrifft Amidin- oder Guanidingruppen-haltige Silane und deren Verwendung als Katalysatoren für die Vernetzung von härtbaren Zusammensetzungen.

### Stand der Technik

Härtbare Zusammensetzungen spielen eine bedeutende Rolle in vielen technischen Anwendungen, beispielsweise als Klebstoffe, Dichtstoffe oder Beschichtungen. Ihre Aushärtung wird durch Vernetzungsreaktionen bewirkt, welche über freie oder latente Reaktivgruppen wie beispielsweise Isocyanatgruppen, Epoxidgruppen, Hydroxylgruppen, Aminogruppen oder Silangruppen ablaufen, wobei diese nach einem Mischvorgang, durch Erwärmen oder durch Kontakt mit Feuchtigkeit mit sich selbst oder untereinander reagieren und so die in der Zusammensetzung enthaltenen Aufbaukomponenten kovalent zu einem polymeren Netzwerk verbinden. Zur Beschleunigung solcher Vernetzungsreaktionen werden häufig Katalysatoren eingesetzt. Sehr oft handelt es sich dabei um toxikologisch bedenkliche Stoffe, welche eine potentielle Gefährdung von Verarbeiter und Umwelt darstellen, insbesondere nach der Aushärtung der Zusammensetzung, wenn der Katalysator oder dessen Abbauprodukte durch Ausgasen, Migration oder Auswaschen freigesetzt werden.

Bei Raumtemperatur härtbare Zusammensetzungen auf Basis von Silangruppen-haltigen Polymeren sind ausgeprägt mit dieser Problemstellung konfrontiert. Silangruppen-haltige Polymere sind dabei insbesondere Polyorganosiloxane, welche gemeinhin als "Silicone" bzw. "Siliconkautschuke" bezeichnet werden, und Silangruppen-haltige organische Polymere, welche auch als "silanfunktionelle Polymere", "silanmodifizierte Polymere" (SMP) oder "silanterminierte Polymere" (STP) bezeichnet werden. Ihre Vernetzung verläuft über die Kondensation von Silanolgruppen unter Ausbildung von Siloxanbindungen und wird klassischerweise mittels Organozinn-Verbindungen, wie insbesondere Di-alkylzinn(IV)carboxylaten, katalysiert. Diese zeichnen sich durch eine sehr hohe Aktivität in Bezug auf die Silanol-Kondensation aus und sind sehr hydrolysebeständig; allerdings sind sie gesundheitsschädlich und stark wassergefährdend. Oft werden sie mit weiteren Katalysatoren kombiniert, hauptsächlich mit basischen Verbindungen wie insbesondere Aminen, welche vor allem die vorgeschaltete Hydrolyse der Silangruppen beschleunigen.

Aufgrund einer stärkeren Gewichtung von EHS-Aspekten durch Berufsverbände und Verbraucher sowie schärferer staatlicher Regulierung werden seit einiger Zeit vermehrt Anstrengungen unternommen, die Organozinn-Verbindungen durch andere, weniger toxische Katalysatoren zu ersetzen. So wurden etwa Organotitanate, -zirkonate und -aluminate als alternative Metall-Katalysatoren beschrieben. Diese haben aber zumeist eine geringere katalytische Aktivität in Bezug auf die Silanol-Kondensation und bewirken eine deutlich langsamere Vernetzung. Wegen ihrer mangelnden Hydrolysestabilität können sie beim Lagern der Zusammensetzung durch Restfeuchte der Inhaltsstoffe einen Grossteil ihrer Aktivität verlieren, wodurch sich die Aushärtung stark verlangsamt oder ganz zum Erliegen kommt.

Eine weitere bekannte Alternative zu Organozinn-Verbindungen stellen stark basische Stickstoff-Verbindungen aus der Klasse der Amidine und Guanidine dar, welche in Kombination mit den erwähnten Metall-Katalysatoren oder auch allein eingesetzt werden können. Viele der gebräuchlichen Amidin- und Guanidin-Katalysatoren, wie insbesondere 1,8-Diazabicyclo[5.4.0]undec-7-en (DBU) und 1,1,3,3-Tetramethylguanidin (TMG), sind allerdings leichtflüchtige und geruchsintensive sowie ebenfalls gesundheitsschädliche und umweltgefährdende Stoffe. Ausserdem neigen sie dazu, aufgrund geringer Verträglichkeit in der Zusammensetzung zu migrieren und dadurch Separation, Ausschwitzen oder Substratverschmutzung zu verursachen. Die beschriebene Verwendung von aromatischen, bei Raumtemperatur festen Amidinen und Guanidinen schafft hier Abhilfe, erfordert aber den Einsatz von geeigneten Lösemitteln und bringt Einbussen bei der katalytischen Aktivität und damit der Vernetzungsgeschwindigkeit.

Das Dokument JP 2002 167438 betrifft die Verwendung von Guanidin modifizierten Silane als Kondensationskatalysatoren.

### Darstellung der Erfindung

Aufgabe der vorliegenden Erfindung ist es daher, einen Katalysator für die Vernetzung von härtbaren Zusammensetzungen, insbesondere Silangruppen-haltigen Zusammensetzungen, bereitzustellen, welcher eine hohe katalytische Aktivität für die Vernetzungsreaktion besitzt und damit eine rasche Aushärtung der applizierten Zusammensetzung ermöglicht, sowie eine hohe Selektivität für diese Vernetzungsreaktion aufweist und somit die Lagerstabilität der Zusammensetzung nicht über Gebühr beeinträchtigt. Weiterhin soll der Katalysator einen geringen Dampfdruck und eine hohe Verträglichkeit mit der Zusammensetzung aufweisen, so dass er weder zu Separierung oder Migration noch zum Verdampfen neigt, und soll möglichst geruchlos und wenig toxisch sein.

Diese Aufgabe wird durch ein Amidin- oder Guanidingruppen-haltiges Silan nach Anspruch 1 gelöst. Das Silan nach Anspruch 1 enthält mindestens einen Alkoxy-Rest mit einer aliphatischen Amidin- oder Guanidingruppe und zeigt eine hohe katalytische Aktivität, während aromatische Amidin- oder Guanidingruppen-haltige Verbindungen kaum oder gar nicht katalytisch aktiv sind. Im Gegensatz zu vielen aus dem Stand der Technik bekannten aliphatischen Amidin- oder Guanidin-Katalysatoren ist es bei Raumtemperatur weitgehend geruchlos und nicht flüchtig. Es zeigt eine hohe kalatytische Aktivität bei guter Selektivität. Dies ist besonders überraschend, würde man doch aufgrund seines relativ hohen Molekulargewichtes eine verminderte Aktivität im Vergleich mit kleineren und somit mobileren Amidinen oder Guanidinen erwarten. Gegenüber dem Einsatz von Hydroxylgruppen aufweisenden Amidinen oder Guanidinen weist es verschiedene Vorteile auf. Die Silane nach Anspruch 1 sind typischerweise flüssig und niedrigviskos, was für deren Verwendung und Dosierung vorteilhaft ist, sie sind insbesondere in über Silangruppen vernetzenden Zusammensetzungen katalytisch aktiver, und sie können während der Lagerung einer solchen Zusammensetzung keine durch freie Hydroxylgruppen verursachten Stabilitätsprobleme auslösen. Zudem kann die bei der Herstellung des Silans nach Anspruch 1 freigesetzte flüchtige organische Verbindung (Alkohol oder Ketoxim) gezielt entfernt werden, was bei der Verwendung in härtbaren Zusammensetzungen vorteilhaft ist, da somit deren Emission von flüchtigen organischen Verbindungen reduziert ist.

Mit diesen Eigenschaften ist das Silan nach Anspruch 1 und/oder ein Amidin- oder Guanidingruppen-haltiges Umsetzungsprodukt davon hervorragend geeignet für die Verwendung in Silangruppen-haltigen Zusammensetzungen, wo es als alleiniger Katalysator oder in Kombination mit weiteren Katalysatoren eine schnelle Aushärtung zu einem mechanisch hochwertigen und beständigen Material ermöglicht, ohne die Lagerfähigkeit der unausgehärteten Zusammensetzung zu beeinträchtigen. Er ist sowohl vor als auch nach der Aushärtung ausgezeichnet verträglich mit der Zusammensetzung und neigt weder zu Separierung noch zu Migration, im Gegensatz zu vielen ähnlichen Zusammensetzungen mit Amidin- und Guanidin-Katalysatoren nach dem Stand der Technik, wo Katalysator-bedingte Migrationseffekte eine starke Rolle spielen. Es ermöglicht emissions- und geruchsarme Produkte, welche weder schmierige oder klebrige Oberflächen aufweisen noch Substratverschmutzung verursachen. Schliesslich ist das Silan nach Anspruch 1 in einem überraschend einfachen und schnellen Verfahren ohne Hilfsstoffe aus handelsüblichen, preisgünstigen Ausgangsmaterialien herstellbar.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Silan der Formel (I), 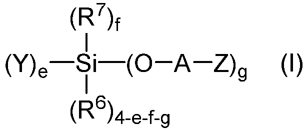 wobei
Z für eine über ein Stickstoffatom gebundene Amidin- oder Guanidin-Gruppe steht,
A für einen zweiwertigen aliphatischen oder cycloaliphatischen oder arylaliphatischen Kohlenwasserstoff-Rest mit 2 bis 20 C-Atomen, welcher gegebenenfalls Ether-Sauerstoff oder sekundären oder tertiären Amin-Stickstoff enthält, steht,
e für 0 oder 1 steht, f für 0 oder 1 oder 2 steht und g für eine ganze Zahl von 1 bis 4 steht, wobei die Summe (e+f+g) für eine ganze Zahl von 1 bis 4 steht,
R⁶ entweder für einen Alkoxy-Rest mit 1 bis 12 C-Atomen, welcher gegebenenfalls Ether-Sauerstoff enthält, oder für einen Ketoximato-Rest mit 1 bis 13 C-Atomen steht,
R⁷ für einen einwertigen Kohlenwasserstoff-Rest mit 1 bis 12 C-Atomen steht, und
Y für einen einwertigen Kohlenwasserstoff-Rest mit 1 bis 20 C-Atomen, welcher gegebenenfalls eine endständige Mercaptogruppe, Epoxidgruppe, (Meth)-acrylgruppe, Amidingruppe, Guanidingruppe, Urethangruppe oder Harnstoffgruppe oder eine endständige Aminogruppe der Formel -NHR⁸ aufweist und welcher gegebenenfalls Ether-Sauerstoff oder sekundären Amin-Stickstoff enthält, steht, wobei R⁸ für einen Wasserstoff-Rest oder einen Alkyl- oder Cycloalkyl- oder Aralkyl-Rest mit 1 bis 8 C-Atomen oder für einen Rest 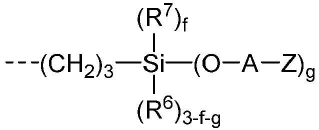 steht,
wobei das Silan der Formel (I) kein Stickstoffatom enthält, welches direkt an einen aromatischen Ring gebunden oder Teil eines heteroaromatischen Ringsystems, wie zum Beispiel Imidazol oder Pyrimidin, ist.

Im vorliegenden Dokument bezeichnet der Begriff "Silangruppe" eine an einen organischen Rest oder an einen Polyorganosiloxan-Rest gebundene Silylgruppe mit einer bis drei, insbesondere zwei oder drei, hydrolysierbaren Substituenten am Silicium-Atom. Besonders gebräuchliche hydrolysierbare Substituenten sind Alkoxy-Reste. Diese Silangruppen werden auch als "Alkoxysilangruppen" bezeichnet. Silangruppen können auch in partiell oder vollständig hydrolysierter Form vorhanden sein.

Als "Hydroxysilan", "Isocyanatosilan", "Aminosilan" bzw. "Mercaptosilan" werden Organoalkoxysilane bezeichnet, die am organischen Rest zusätzlich zur Silangruppe eine oder mehrere Hydroxyl-, Isocyanato-, Amino- bzw. Mercaptogruppen aufweisen.

Als "primäre Aminogruppe" bzw. "primärer Amin-Stickstoff" wird eine NH₂-Gruppe bzw. deren Stickstoffatom bezeichnet, die bzw. das an einen organischen Rest gebunden ist, und als "sekundäre Aminogruppe" bzw. "sekundärer Amin-Stickstoff" wird eine NH-Gruppe bzw. deren Stickstoffatom bezeichnet, die bzw. das an zwei organische Reste, welche auch gemeinsam Teil eines Rings sein können, gebunden ist, und als "tertiäre Aminogruppe" bzw. "tertiärer Amin-Stickstoff" wird eine N-Gruppe bzw. deren Stickstoffatom bezeichnet, die bzw. das an drei organische Reste, welche auch zu zweit oder zu dritt Teil eines oder mehrerer Ringe sein können, gebunden ist.

Mit "Poly" beginnende Substanznamen wie Polyol oder Polyisocyanat bezeichnen Substanzen, die formal zwei oder mehr der in ihrem Namen vorkommenden funktionellen Gruppen pro Molekül enthalten.

Der Begriff "organisches Polymer" umfasst ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde und im PolymerRückgrat mehrheitlich Kohlenstoffatome aufweist, sowie Umsetzungsprodukte eines solchen Kollektivs von Makromolekülen. Polymere mit einem Polyorganosiloxan-Rückgrat (gemeinhin als "Silicone" bezeichnet) stellen keine organischen Polymere im Sinne des vorliegenden Dokuments dar.

Der Begriff "Silangruppen-haltiger Polyether" umfasst auch Silangruppen-haltige organische Polymere, welche zusätzlich zu Polyether-Einheiten auch Urethangruppen, Harnstoffgruppen oder Thiourethangruppen enthalten können. Solche Silangruppen-haltige Polyether können auch als "Silangruppen-haltige Polyurethane" bezeichnet werden.

Unter "Molekulargewicht" versteht man im vorliegenden Dokument die molare Masse (in Gramm pro Mol) eines Moleküls oder eines Teils eines Moleküls, auch als "Rest" bezeichnet. Als "mittleres Molekulargewicht" wird das Zahlenmittel Mₙ einer oligomeren oder polymeren Mischung von Molekülen oder Resten bezeichnet, welches üblicherweise mittels Gelpermeationschromatographie (GPC) gegen Polystyrol als Standard bestimmt wird.

Als "lagerstabil" oder "lagerfähig" wird eine Substanz oder eine Zusammensetzung bezeichnet, wenn sie bei Raumtemperatur in einem geeigneten Gebinde während längerer Zeit, typischerweise mindestens 3 Monaten bis zu 6 Monaten und mehr, aufbewahrt werden kann, ohne dass sie sich in ihren Anwendungs- oder Gebrauchseigenschaften, insbesondere der Viskosität und der Vernetzungsgeschwindigkeit, durch die Lagerung in einem für ihren Gebrauch relevanten Ausmass verändert.

Eine gestrichelte Linie in den Formeln in diesem Dokument stellt jeweils die Bindung zwischen einem Substituenten und dem zugehörigen Molekülrest dar. Als "Raumtemperatur" wird eine Temperatur von ca. 23 °C bezeichnet.

Das Silan der Formel (I) kann auch in tautomerer Form vorliegen. Alle möglichen Tautomerformen des Silans der Formel (I) werden im Rahmen der vorliegenden Erfindung als gleichwertig angesehen.

Weiterhin kann das Silan der Formel (I) in protonierter Form vorliegen. Ebenfalls kann das Silan der Formel (I) in komplexierter Form vorliegen, insbesondere mit Kationen von Zink, Eisen oder Molybdän. Bevorzugt steht Z für 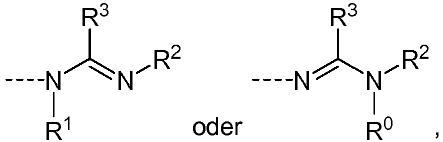 wobei
R⁰ für einen Wasserstoff-Rest oder für einen Alkyl- oder Cycloalkyl- oder Aralkyl-Rest mit 1 bis 8 C-Atomen steht,
R¹ für einen Wasserstoff-Rest oder für einen Alkyl- oder Cycloalkyl- oder Aralkyl-Rest mit 1 bis 8 C-Atomen steht oder zusammen mit R² für R⁹ steht,
R² für einen Wasserstoff-Rest oder für einen Alkyl-, Cycloalkyl- oder Aralkyl-Rest mit 1 bis 18 C-Atomen, welcher gegebenenfalls Ether-Sauerstoff oder tertiären Amin-Stickstoff enthält, oder zusammen mit R¹ für R⁹ steht,
R³ für -NR⁴R⁵ oder für einen Wasserstoff-Rest oder für einen Alkyl- oder Cycloalkyl- oder Aralkyl-Rest mit 1 bis 12 C-Atomen steht,
R⁴ und R⁵ unabhängig voneinander jeweils für einen Wasserstoff-Rest oder für einen Alkyl-, Cycloalkyl- oder Aralkyl-Rest mit 1 bis 18 C-Atomen, welcher gegebenenfalls Ether-Sauerstoff oder tertiären Amin-Stickstoff enthält, stehen, und
R⁹ für 1,2-Ethylen oder 1,2-Propylen oder 1,3-Propylen oder 1,3-Butylen oder 1,3-Pentylen steht,
   wobei
R² und R⁰ auch zusammen für einen Alkylen-Rest mit 3 bis 6 C-Atomen, welcher gegebenenfalls Ether-Sauerstoff oder tertiären Amin-Stickstoff enthält, stehen können,
R² und R³ auch zusammen für einen Alkylen-Rest mit 3 bis 6 C-Atomen stehen können,
R⁴ und R⁵ auch zusammen für einen Alkylen-Rest mit 4 bis 7 C-Atomen, welcher gegebenenfalls Ether-Sauerstoff oder tertiären Amin-Stickstoff enthält enthält, stehen können, und
R² und R⁵ auch zusammen für einen Alkylen-Rest mit 2 bis 12 C-Atomen stehen können.

Bevorzugt steht A für einen Alkylen-Rest mit 2 bis 10, insbesondere 2 bis 6, C-Atomen, welcher gegebenenfalls ein oder zwei Ether-Sauerstoffe enthält.

Insbesondere ist A ausgewählt aus der Gruppe bestehend aus 1,2-Ethylen, 1,2-Propylen, 1,3-Propylen, 1,4-Butylen, 1,1-Dimethyl-1,2-ethylen, 1,5-Pentylen, 1,6-Hexylen, (1,5,5-Trimethylcyclohexan-1-yl)methan-1,3, 3-Oxa-1,5-pentylen und 3,6-Dioxa-1,8-octylen.

Bevorzugt steht e für 0 oder 1.

Bevorzugt steht f für 0 oder 1, insbesondere für 0.
Bevorzugt steht R⁶ für einen Akoxy-Rest mit 1 bis 4 C-Atomen, welcher gegebenenfalls ein oder zwei Ether-Sauerstoffe enthält, insbesondere für Methoxy oder Ethoxy.
Weiterhin bevorzugt steht R⁶ für einen Ketoximato-Rest mit 1 bis 6 C-Atomen, insbesondere für Methylethyketoximato oder Methylisobutylketoximato. Bevorzugt steht R⁷ für einen einwertigen Kohlenwasserstoff-Rest mit 1 bis 6 C-Atomen, insbesondere für einen Methyl-Rest oder für einen Phenyl-Rest.

Bevorzugt steht Y für einen einwertigen Kohlenwasserstoff-Rest mit 1 bis 8 C-Atomen, welcher gegebenenfalls eine endständige Mercaptogruppe, Epoxidgruppe, (Meth)acrylgruppe, Amidingruppe, Guanidingruppe, Urethangruppe oder Harnstoffgruppe oder eine endständige Aminogruppe der Formel -NHR⁸ aufweist, und welcher gegebenenfalls einen Ether-Sauerstoff oder einen sekundären Amin-Stickstoff enthält.

Insbesondere ist Y ausgewählt aus der Gruppe bestehend aus Methyl, Octyl, Isooctyl, Phenyl, Vinyl, 3-Aminopropyl, N-(2-Aminoethyl)-3-aminopropyl, 3-Mercaptopropyl, 3-Glycidoxypropyl, 3-Acryloxypropyl, 3-Methacryloxypropyl und einem Rest der Formel 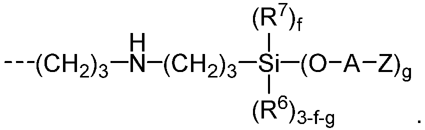
R⁰ steht bevorzugt für einen Wasserstoff-Rest oder für einen Alkyl-Rest mit 1 bis 4 C-Atomen, insbesondere für einen Wasserstoff-Rest.
R¹ steht bevorzugt für einen Wasserstoff-Rest oder für einen Alkyl- oder Cycloalkyl- oder Aralkyl-Rest mit 1 bis 4 C-Atomen oder zusammen mit R² für R⁹. R² steht bevorzugt für einen Alkyl-, Cycloalkyl- oder Aralkyl-Rest mit 1 bis 12, insbesondere 1 bis 8, C-Atomen, welcher gegebenenfalls Ether-Sauerstoff oder tertiären Amin-Stickstoff enthält, oder zusammen mit R¹ für R⁹.
R³ steht bevorzugt für -NR⁴R⁵ oder für einen Wasserstoff-Rest oder für einen Alkyl-, Cycloalkyl- oder Aralkyl-Rest mit 1 bis 8, insbesondere 1 bis 4, C-Atomen.
R⁴ und R⁵ stehen bevorzugt unabhängig voneinander jeweils für einen Wasserstoff-Rest oder für einen Alkyl-, Cycloalkyl- oder Aralkyl-Rest mit 1 bis 12 C-Atomen, welcher gegebenenfalls einen Ether-Sauerstoff oder einen tertiären Amin-Stickstoff enthält.
Weiterhin bevorzugt stehen R⁴ und R⁵ zusammen für einen Alkylen-Rest mit 4 bis 7 C-Atomen, welcher gegebenenfalls einen Ether-Sauerstoff oder einen tertiären Amin-Stickstoff enthält.
Besonders bevorzugt steht R⁴ für einen Wasserstoff-Rest.

In einer bevorzugten Ausführungsform steht Z für eine Amidin-Gruppe. Ein solches Silan wird im Folgenden auch als "Amidinosilan der Formel (I)" bezeichnet.
R³ steht dabei für einen Wasserstoff-Rest oder für einen Alkyl-, Cycloalkyl- oder Aralkyl-Rest mit 1 bis 12, bevorzugt 1 bis 8, insbesondere 1 bis 4, C-Atomen, oder zusammen mit R² für einen Alkylen-Rest mit 3 bis 6, insbesondere 3 bis 5, C-Atomen.
Besonders bevorzugt steht R³ für einen Wasserstoff-Rest oder für einen Methyl-Rest, insbesondere für einen Methyl-Rest.
Ein Amidinosilan der Formel (I) weist den Vorteil auf, dass es eine nicht ganz so hohe katalytische Aktivität aufweist und deshalb in etwas höherer Menge eingesetzt werden kann, wodurch es weniger anfällig ist für Störungen durch andere Bestandteile der Zusammensetzung, insbesondere den darin enthaltenen Verunreinigungen.

In einem bevorzugten Amidinosilan der Formel (I) steht Z für 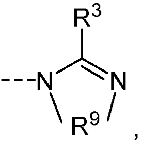 wobei R³ und R⁹ die genannten Bedeutungen aufweisen.
Besonders bevorzugt steht R³ für einen Wasserstoff-Rest oder für einen Methyl-Rest.
Bevorzugt steht R⁹ für 1,3-Propylen.
Meist bevorzugt steht R³ für Methyl und R⁹ für 1,3-Propylen.

In einer weiteren bevorzugten Ausführungsform steht Z für eine Guanidin-Gruppe. Ein solches Silan wird im Folgenden auch als "Guanidinosilan der Formel (I)" bezeichnet.
Dabei steht R³ für-NR⁴R⁵.
Bevorzugt steht R⁴ für einen Wasserstoff-Rest.

Bevorzugt steht R⁵ für einen Alkyl-, Cycloalkyl- oder Aralkyl-Rest mit 1 bis 18 C-Atomen, welcher gegebenenfalls Heteroatome enthält.
Ein Guanidinosilan der Formel (I) weist den Vorteil auf, dass es eine besonders hohe katalytische Aktivität aufweist.

In einem bevorzugten Guanidinosilan der Formel (I) steht Z für 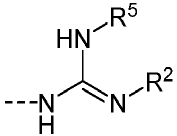 , wobei R² und R⁵ unabhängig voneinander jeweils für einen Alkyl-, Cycloalkyl- oder Aralkyl-Rest mit 1 bis 12 C-Atomen, welcher gegebenenfalls Ether-Sauerstoff oder tertiären Amin-Stickstoff enthält, stehen.
Bevorzugt stehen R² und R⁵ dabei unabhängig voneinander jeweils für Ethyl, Isopropyl, tert.Butyl, 3-(Dimethylamino)propyl oder Cyclohexyl, insbesondere für Isopropyl oder Cyclohexyl.

Die bevorzugten Silane der Formel (I) lassen sich aus handelsüblichen, preisgünstigen Rohstoffen einfach herstellen und zeigen eine hohe katalytische Aktivität und Verträglichkeit in härtbaren Zusammensetzungen, insbesondere solchen auf Basis von Silangruppen-haltigen Polymeren.

Ein weiterer Gegenstand der Erfindung ein Verfahren zur Herstellung des Silans der Formel (I), wobei
- mindestens ein Amin der Formel (IIa) oder (IIb),

   HO-A-NHR¹ (IIa)

   HO-A-NH-R⁹-NH₂ (IIb)
- gegebenenfalls mindestens ein Amin der Formel R²-NH-R⁰, wobei
   R⁰ für einen Wasserstoff-Rest oder für einen Alkyl- oder Cycloalkyl- oder Aralkyl-Rest mit 1 bis 8 C-Atomen und
   R² für einen Alkyl-, Cycloalkyl- oder Aralkyl-Rest mit 1 bis 18 C-Atomen, welcher gegebenenfalls Heteroatome enthält, oder
   R⁰ und R² zusammen für einen Alkylen-Rest mit 3 bis 6 C-Atomen, welcher gegebenenfalls Heteroatome enthält, stehen,
- mindestens ein Reagens zur Einführung von Amidin- oder Guanidin-Gruppen und
- mindestens ein Alkoxy- oder Ketoximato-Silan miteinander umgesetzt werden.
A, R¹, und R⁹ weisen dabei die bereits genannten Bedeutungen auf.

Das Reaktionsprodukt aus diesem Verfahren kann ohne weitere Aufarbeitung als Katalysator für die Vernetzung einer härtbaren Zusammensetzung verwendet werden.

Als Amin der Formel (IIa) oder (IIb) geeignet sind aliphatische oder cycloaliphatische oder arylaliphatische Hydroxylamine, insbesondere 2-Aminoethanol, 2-Methylaminoethanol (2-Amino-1-propanol), 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol, eine primäre Aminogruppe tragende Derivate von Glykolen wie Diethylenglykol, Dipropylenglykol, Dibutylenglykol oder höheren Oligomeren oder Polymeren dieser Glykole, insbesondere 2-(2-Aminoethoxy)ethanol, 2-(2-(2-Aminoethoxy)ethoxy)ethanol, α-(2-Hydroxymethylethyl)-ω-(2-aminomethylethoxy)poly(oxy(methyl-1,2-ethandiyl)), eine Hydroxylgruppe und eine primäre Aminogruppe tragende Derivate von polyalkoxylierten drei- oder höherwertigen Alkoholen, Produkte aus der einfachen Cyanoethylierung und anschliessenden Hydrierung von Glykolen, insbesondere 3-(2-Hydroxyethoxy)propylamin, 3-(2-(2-Hydroxyethoxy)ethoxy)-propylamin oder 3-(6-Hydroxyhexyloxy)propylamin, sowie weiterhin Hydroxylamine mit einer primären und einer sekundären Aminogruppe wie insbesondere N-(2-Aminoethyl)-2-aminoethanol oder N-(3-Aminopropyl)-2-aminoethanol.

Bevorzugt ist das Amin der Formel (IIa) oder (IIb) ausgewählt aus der Gruppe bestehend aus 2-Aminoethanol, 1-Amino-2-propanol, 3-Amino-1-propanol, 4-Amino-1-butanol, 2-Amino-2-methylpropanol, 5-Amino-1-pentanol, 6-Amino-1-hexanol, 3-Aminomethyl-3,5,5-trimethyl-cyclohexanol, 2-(2-Aminoethoxy)ethanol, 2-(2-(2-Aminoethoxy)ethoxy)ethanol, N-(2-Aminoethyl)-2-aminoethanol und N-(3-Aminopropyl)-2-aminoethanol.

Als Amin der Formel R²-NH-R⁰ geeignet sind aliphatische, cycloaliphatische oder arylaliphatische Monoamine, insbesondere Amine der Formel R²-NH₂. Bevorzugt sind Methylamin, Ethylamin, Propylamin, Isopropylamin, n-Butylamin, Isobutylamin, sec.Butylamin, n-Hexylamin, Cyclohexylamin, Benzylamin, 2-Ethylhexylamin, n-Octylamin, n-Decylamin, Laurylamin oder 2-Methoxyethylamin.

Das Reagens zur Einführung von Amidin- oder Guanidin-Gruppen ist bevorzugt ausgewählt aus der Gruppe bestehend aus Orthoestern, 1,3-Ketoestern, 1,3-Ketoamiden, Nitrilen, Imidsäureestern, Imidsäurechloriden, Amiden, Lactamen, Cyanamiden, Carbodiimiden, Harnstoffen, O-Alkylisoharnstoffen, Thioharnstoffen, S-Alkylisothioharnstoffen, Aminoiminomethansulfonsäuren, Guanylpyrazolen und Guanidinen.

Zur Einführung von Amidin-Gruppen geeignet sind Orthoester, 1,3-Ketoester, 1,3-Ketoamide, Nitrile, Imidsäureester, Imidsäurechloride, Amide oder Lactame. Bevorzugt sind Orthoester, 1,3-Ketoester oder Nitrile.
Bevorzugte Orthoester sind Orthoester der Formel R³-C(OR^{a})₃, wobei R³ die genannten Bedeutungen aufweist und R^{a} für einen Alkyl-Rest mit 1 bis 4 C-Atomen steht. Besonders geeignet ist ein Orthoformiat, Orthoacetat, Orthopropionat, Orthobutyrat oder Orthovalerat, insbesondere Trimethylorthoformiat, Triethylorthoformiat, Trimethylorthoacetat oder Triethylorthoacetat. Bevorzugte 1,3-Ketoester sind 1,3-Ketoester der Formel R³-C(O)CH₂C(O)OR^{a}, wobei R³ und R^{a} die bereits genannten Bedeutungen aufweisen, insbesondere Methylacetoacetat, Ethylacetoacetat, Isopropylacetoacetat oder tert.Butylacetoacetat, besonders bevorzugt Ethylacetoacetat.

Bevorzugte Nitrile sind Nitrile der Formel R³-CN, wobei R³ die genannten Bedeutungen aufweist, insbesondere Acetonitril, Propionitril, Butyronitril, Isobutyronitril, Valeronitril oder Capronitril, besonders bevorzugt Acetonitril.

Zur Einführung von Guanidin-Gruppen geeignet sind Cyanamide, Carbodimide, Harnstoffe, O-Alkylisoharnstoffe, Thioharnstoffe, S-Alkylisothioharnstoffe, Aminoiminomethansulfonsäuren, Guanylpyrazole oder Guanidine. Bevorzugt sind Cyanamide oder Carbodiimide, insbesondere Carbodiimide.

Als Carbodiimid bevorzugt sind Carbodiimide der Formel R⁵N=C=NR², wobei R² und R⁵ die bereits genannten Bedeutungen aufweisen. Besonders bevorzugt ist N,N'-Diisopropylcarbodiimid (DIC), N,N'-Di-tert.butylcarbodiimid, N,N'-Dicyclohexylcarbodiimid (DCC) oder N-Ethyl-N'-(3-dimethylaminopropyl)carbodiimid (EDC), insbesondere N,N'-Diisopropylcarbodiimid (DIC) oder N,N'-Dicyclohexylcarbodiimid (DCC).

Bevorzugt ist das Reagens zur Einführung von Amidin- oder Guanidin-Gruppen ausgewählt aus der Gruppe bestehend aus Trimethylorthoformiat, Triethylorthoformiat, Trimethylorthoacetat, Triethylorthoacetat, Methylacetoacetat, Ethylacetoacetat, Isopropylacetoacetat, tert.Butylacetoacetat, Acetonitril, N,N'-Diisopropylcarbodiimid, N,N'-Di-tert.butylcarbodiimid, N-Ethyl-N'-(3-dimethylaminopropyl)carbodiimid und N,N'-Dicyclohexylcarbodiimid.
Mit diesen Reagentien werden auf besonders einfache Weise Silane mit besonders hoher katalytischer Aktivität erhalten.

Bevorzugte Alkoxy- oder Ketoximato-Silane sind Orthosilicate, Organoalkoxysilane, Organoketoximatosilane oder Silane mit Alkoxy- und Ketoximato-Gruppen.
Bevorzugt ist das Alkoxy- oder Ketoximato-Silan ausgewählt aus der Gruppe bestehend aus Tetramethylorthosilicat, Tetraethylorthosilicat, Methyltrimethoxysilan, Dimethyldimethoxysilan, Ethyltrimethoxysilan, Propytrimethoxysilan, Phenyltrimethoxysilan, Phenyldimethoxymethylsilan, Octyltrimethoxysilan, Isooctyltrimethoxysilan, Vinyltrimethoxysilan, Vinyldimethoxymethylsilan, 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropyldimethoxymethylsilan, N,N-Bis(trimethoxysilylpropyl)amin, 3-Mercaptopropyltrimethoxysilan, 3-Mercaptopropyldimethoxymethylsilan, 3-Glycidoxypropyltrimethoxysilan, 3-Glycidoxypropyldimethoxymethylsilan, 3-Acryloxypropyltrimethoxysilan, 3-Acryloxypropyldimethoxymethylsilan, 3-Methacryloxypropyltrimethoxysilan, 3-Methacryloxypropyldimethoxymethylsilan, Methyltris(methylethylketoximato)silan, Vinyltris(methylethylketoximato)silan, Methyltris(methylisobutylketoximato)silan, Vinyltris(methylisobutylketoximato)silan und die entsprechenden Organoalkoxysilane mit Ethoxy- anstelle der Methoxygruppen.

Die Umsetzung kann einstufig oder mehrstufig erfolgen. Bevorzugt erfolgt sie zweistufig.

Eine bevorzugte Ausführungsform des Verfahren zur Herstellung des Silans der Formel (I) besteht darin, dass
in einem ersten Schritt das Amin der Formel (IIa) oder (IIb) und gegebenenfalls das Amin der Formel R²-NH-R⁰ mit dem Reagens zur Einführung von Amidin- oder Guanidin-Gruppen zu einem Hydroxy-Amidin oder -Guanidin der Formel (III) umgesetzt wird,

HO-A-Z (III)

und
in einem zweiten Schritt das Hydroxy-Amidin oder -Guanidin der Formel (III) mit dem Alkoxysilan zum Silan der Formel (I) umgesetzt wird.
A und Z weisen dabei die bereits genannten Bedeutungen auf.

Die Umsetzung im ersten Schritt des beschriebenen Verfahrens wird bevorzugt bei erhöhter Temperatur, gegebenenfalls unter erhöhtem Druck und gegebenenfalls in Gegenwart eines Katalysators, durchgeführt, wobei aus dem Reagens zur Einführung von Amidin- oder Guanidin-Gruppen freigesetzte Abspalter wie Alkohole, Ester oder Amine während oder nach der Umsetzung bevorzugt entfernt werden, insbesondere mittels Destillation, gegebenenfalls unter Vakuum.
Wird als Reagens zur Einführung von Amidin-Gruppen ein Orthoester der Formel R³-C(OR^{a})₃ eingesetzt, so erfolgt die Umsetzung bevorzugt bei einer Temperatur von 40 bis 160 °C, insbesondere 60 bis 140 °C, wobei der freigesetzte Alkohol R^{a}OH bevorzugt destillativ entfernt wird. Gegebenenfalls wird dabei ein Katalysator eingesetzt, insbesondere eine Säure.
Wird als Reagens zur Einführung von Amidin-Gruppen ein 1,3-Ketoester der Formel R³⁻C(O)CH₂C(O)OR^{a} eingesetzt, so erfolgt die Umsetzung bevorzugt bei einer Temperatur von 20 bis 100 °C, insbesondere 40 bis 80 °C, wobei der freigesetzte Ester CH₃C(O)OR^{a} bevorzugt destillativ entfernt wird. Bevorzugt wird dabei ein Katalysator eingesetzt, insbesondere eine Säure, bevorzugt eine Sulfonsäure.
Wird als Reagens zur Einführung von Amidin-Gruppen ein Nitril der Formel R³-CN eingesetzt, so erfolgt die Umsetzung bevorzugt bei einer Temperatur von 60 bis 180 °C, insbesondere 80 bis 160 °C, gegebenenfalls unter erhöhtem Druck, wobei der freigesetzte Ammoniak bevorzugt destillativ entfernt wird. Bevorzugt wird dabei ein Katalysator eingesetzt, insbesondere eine Lewissäure, bevorzugt Bortrifluorid-Etherat, Lithiumperchlorat, Zinkchlorid, Zinktriflat oder Lanthantriflat.
Wird als Reagens zur Einführung von Guanidin-Gruppen ein Carbodiimid der Formel R⁵N=C=NR² eingesetzt, so erfolgt die Umsetzung bevorzugt bei einer Temperatur von 40 bis 160 °C, insbesondere 60 bis 140 °C. Gegebenenfalls wird dabei ein Katalysator eingesetzt, insbesondere eine Säure, bevorzugt eine Carbonsäure oder eine Lewissäure, besonders bevorzugt Bortrifluorid-Etherat, Lithiumperchlorat, Zinkchlorid, Zinktriflat oder Lanthantriflat.
Bevorzugt wird das Verhältnis zwischen dem Amin der Formel (IIa) oder (IIb) und dem Reagens zur Einführung von Amidin- oder Guanidin-Gruppen so gewählt, dass das Reagens zur Einführung von Amidin- oder Guanidin-Gruppen bei der Umsetzung vollständig umgesetzt wird.

Die Umsetzung im zweiten Schritt des beschriebenen Verfahrens wird bevorzugt bei einer Temperatur im Bereich von 20 bis 160 °C durchgeführt, wobei die durch Umesterung aus dem Alkoxy- oder Ketoximato-Silan freigesetzten Alkohole oder Ketoxime während oder nach der Umsetzung aus der Reaktionsmischung bevorzugt entfernt werden, insbesondere mittels Destillation, gegebenenfalls unter Vakuum. Gegebenenfalls wird dabei ein Katalysator eingesetzt, der die Umesterungsreaktion des Alkoxy- oder Ketoximato-Silans beschleunigt.
Bevorzugt wird das Verhältnis zwischen dem Hydroxy-Amidin oder -Guanidin der Formel (III) und dem Alkoxy- oder Ketoximato-Silan so gewählt, dass höchstens eine Hydroxylgruppe des Hydroxy-Amidins oder -Guanidins auf eine Alkoxy- oder Ketoximato-Gruppe vorhanden ist. Besonders bevorzugt liegt das Molverhältnis zwischen dem Hydroxy-Amidin oder -Guanidin der Formel (III) und dem Alkoxy- oder Ketoximato-Silan ungefähr bei 1:1.

Als Hydroxy-Amidin der Formel (III) bevorzugt sind Umsetzungsprodukte aus einem Amin der Formel (IIa) oder (IIb) und gegebenenfalls einem Amin der Formel R²-NH-R⁰ mit einem Orthoester der Formel R³-C(OR^{a})₃ oder mit einem 1,3-Ketoester der Formel R³-C(O)CH₂C(O)OR^{a} oder mit einem Nitril der Formel R³-CN.
Als Hydroxy-Guanidin der Formel (III) bevorzugt sind Umsetzungsprodukte aus einem Amin der Formel (IIa) oder (IIb) mit einem Carbodiimid der Formel R⁵N=C=NR².

Besonders bevorzugt ist das Hydroxy-Amidin oder -Guanidin der Formel (III) ausgewählt aus der Gruppe bestehend aus 1-(2-Hydroxypropyl)-2,3-diisopropylguanidin, 1-(2-Hydroxypropyl)-2,3-dicyclohexylguanidin, 1-(3-Hydroxypropyl)-2,3-diisopropylguanidin, 1-(3-Hydroxypropyl)-2,3-dicyclohexylguanidin, 1-(4-Hydroxybutyl)-2,3-diisopropylguanidin, 1-(4-Hydroxybutyl)-2,3-dicyclohexylguanidin, 1-(2-Hydroxy-1,1-dimethylethyl)-2,3-diisopropylguanidin, 1-(2-Hydroxy-1,1 -dimethylethyl)-2,3-dicyclohexylguanidin, 1-(5-Hydroxypentyl)-2,3-diisopropylguanidin, 1-(5-Hydroxypentyl)-2,3-dicyclohexylguanidin, 1-(6-Hydroxyhexyl)-2,3-diisopropylguanidin, 1-(6-Hydroxyhexyl)-2,3-dicyclohexylguanidin, 1-(3-Hydroxy-1,5,5-trimethylcyclohexylmethyl)-2,3-diisopropylguanidin, 1-(3-Hydroxy-1,5,5-trimethylcyclohexylmethyl)-2,3-dicyclohexylguanidin, 1-(2-(2-Hydroxyethoxy)ethyl)-2,3-diisopropylguanidin, 1-(2-(2-Hydroxyethoxy)ethyl)-2,3-dicyclohexylguanidin, 1-(2-(2-(2-Hydroxyethoxy)ethoxy)ethyl)-2,3-diisopropylguanidin, 1-(2-(2-(2-Hydroxyethoxy)ethoxy)ethyl)-2,3-dicyclohexylguanidin, 1-(2-Hydroxyethyl)-2-methyl-imidazolin und 1-(2-Hydroxyethyl)-2-methyl-1,4,5,6-tetrahydropyrimidin.

Eine weitere bevorzugte Ausführungsform des Verfahren zur Herstellung des Silans der Formel (I) besteht darin, dass
in einem ersten Schritt das Amin der Formel (IIa) oder (IIb) mit dem Alkoxy- oder Ketoximato-Silan zu einem Silan der Formel (IVa) oder (IVb) umgesetzt wird, 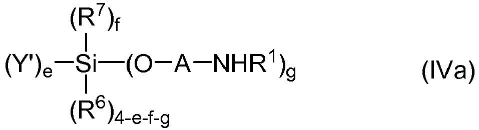 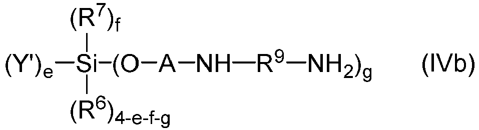 wobei
Y' für einen einwertigen Kohlenwasserstoff-Rest mit 1 bis 20 C-Atomen, welcher gegebenenfalls eine endständige Mercaptogruppe, Epoxidgruppe, (Meth)acrylgruppe, Amidingruppe, Guanidingruppe, Urethangruppe oder Harnstoffgruppe oder eine endständige Aminogruppe der Formel -NHR⁸ aufweist und welcher gegebenenfalls Ether-Sauerstoff oder sekundären Amin-Stickstoff enthält, steht,
wobei R^{8'} für einen Wasserstoff-Rest oder einen Alkyl- oder Cycloalkyl- oder Aralkyl-Rest mit 1 bis 8 Kohlenstoffatomen oder für einen Rest der Formel 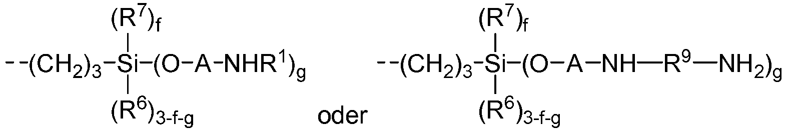 steht,
und e, f, g, A, R¹, R⁶, R⁷ und R⁹ die genannten Bedeutungen aufweisen, und
in einem zweiten Schritt das Silan der Formel (IVa) oder (IVb) mit dem Reagens zur Einführung von Amidin- oder Guanidin-Gruppen zum Silan der Formel (I) umgesetzt wird.

Die Reaktionsbedingungen für die an den jeweiligen Umsetzungen beteiligten Reagentien sind dabei bevorzugt dieselben wie für das beschriebene Verfahren via das Hydroxy-Amidin oder -Guanidin der Formel (III), wobei die durch Umesterung aus dem Alkoxy- oder Ketoximato-Silan freigesetzten Alkohole oder Ketoxime bevorzugt ebenfalls während oder nach der Umsetzung aus der Reaktionsmischung entfernt werden.

Ein weiterer Gegenstand der Erfindung ist ein Amidin- oder Guanidingruppen-haltiges Umsetzungsprodukt, welches aus einem Silan der Formel (I), bei welchem (e+f+g) für 1 oder 2 oder 3 steht, durch Kondensation mit mindestens einer Silanolgruppen-haltigen Verbindung erhalten wird. Ein solches Umsetzungsproukt kann auf die gleiche Weise wie das Silan der Formel (I) als Katalysator verwendet werden. Bevorzugt sind bei Raumtemperatur flüssige Umsetzungsprodukte.

In einer Ausführungsform wird ein solches Umsetzungsprodukt allein aus dem Silan der Formel (I), bei welchem (e+f+g) für 1 oder 2 oder 3 steht, durch Hydrolyse- und nachfolgende Kondensationsreaktionen erhalten und stellt ein Silanol- und/oder Siloxangruppen aufweisendes oligomeres Folgeprodukt des Silans dar.

In einer weiteren Ausführungsform wird ein solches Umsetzungsprodukt erhalten aus der Kondensation mit mindestens einem Siliconöl der Formel 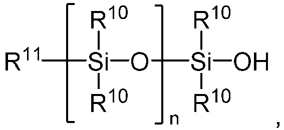 wobei
n für eine ganze Zahl im Bereich von 3 bis 200, bevorzugt 5 bis 80, insbesondere 5 bis 20, steht,
R¹⁰ für einen einwertigen Kohlenwasserstoffrest mit 1 bis 6 C-Atomen steht, und
R¹¹ für einen Hydroxyl-Rest oder für einen Alkyl- oder Alkoxy- oder Ketoximato-Rest mit 1 bis 12, insbesondere 1 bis 6, C-Atomen steht.
Bevorzugt weist das Siliconöl ein mittleres Molekulargewicht im Bereich von 312 bis 15'000 g/mol, insbesondere 460 bis 6'000 g/mol, auf.
Besonders bevorzugt sind Siliconöle mit einem mittleren Molekulargewicht im Bereich von ungefähr 500 bis ungefähr 1'500 g/mol.

Die Kondensation wird bevorzugt bei einer Temperatur im Bereich von 20 bis 160 °C durchgeführt, gegebenenfalls in Anwesenheit geeigneter Katalysatoren, wobei der freigesetzte Abspalter HR⁶ (ein Alkohol oder Ketoxim) während oder nach der Umsetzung aus der Reaktionsmischung entfernt werden kann. Bevorzugt ist dabei ein Verhältnis zwischen dem Silan der Formel (I) und den Silanolgruppen des Siliconöls von ungefähr 1:1.

Ein solches Umsetzungsprodukt weist insbesondere die Formel (la) auf, 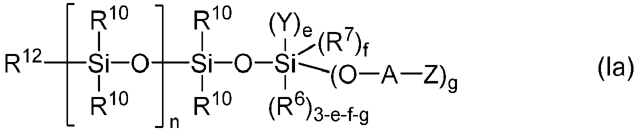 wobei
R¹² entweder für einen Hydroxyl-Rest oder für einen Alkyl- oder Alkoxy- oder Ketoximato-Rest mit 1 bis 12, insbesondere 1 bis 6, C-Atomen oder für einen Rest 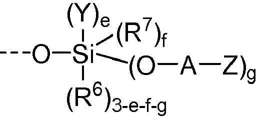 steht,
und e, f, g, n, R⁶, R⁷, R¹⁰, A und Z die bereits genannten Bedeutungen aufweisen.

Ebenfalls möglich ist die Herstellung eines Umsetzungsprodukts der Formel (la), indem das Siliconöl zuerst mit mindestens einem Alkoxy- oder Ketoximato-Silan kondensiert wird und das erhaltene Produkt anschliessend mit mindestens einem Hydroxy-Amidin oder -Guanidin der Formel (III), bei welchem (e+f+g) für 1 oder 2 oder 3 steht, umgeestert wird.

Das Silan der Formel (I) und/oder ein Amidin- oder Guanidingruppen-haltiges Umsetzungsprodukt davon zeichnet sich insbesondere dadurch aus, dass es eine beschleunigende Wirkung ausübt auf Reaktivgruppen, beispielsweise Isocyanatgruppen, Epoxidgruppen, Hydroxylgruppen, Aminogruppen oder Silangruppen, wie sie in härtbaren Zusammensetzungen enthalten sind und zu Vernetzungsreaktionen mit sich selbst oder untereinander fähig sind. Es weist insbesondere eine katalytische Aktivität in Bezug auf die Hydrolyse und Kondensationsreaktion von Silangruppen auf. Es eignet sich somit insbesondere als Katalysator zur Beschleunigung der Vernetzung von härtbaren Zusammensetzungen, insbesondere solchen auf Basis von Silangruppen-haltigen Polymeren. Da das Silan der Formel (I) und/oder ein Amidin- oder Guanidingruppen-haltiges Umsetzungsprodukt davon weiterhin einen niedrigen Dampfdruck aufweist, bevorzugt flüssig ist, eine gute Verträglichkeit mit vielen Silangruppen-haltigen Polymeren zeigt und deren Lagerstabilität nicht negativ beeinflusst, ermöglicht es insbesondere die Formulierung von besonders emissions- und geruchsarmen Produkten, welche nicht zu Separierung oder Migration des Katalysators neigen.

Dementsprechend betrifft die Erfindung auch die Verwendung des Silans der Formel (I) oder eines Amidin- oder Guanidingruppen-haltigen Umsetzungsproduktes davon als Katalysator in härtbaren Zusammensetzungen, insbesondere Silangruppen-haltigen Zusammensetzungen, wo es die Vernetzung bzw. Aushärtung der Zusammensetzung beschleunigt.

Als härtbare Zusammensetzung bevorzugt sind Zusammensetzungen enthaltend mindestens ein Silangruppen-haltiges Polymer, wobei dieses insbesondere ausgewählt ist aus der Gruppe besehend aus Polyorganosiloxanen mit endständigen Silangruppen und Silangruppen-haltigen organischen Polymeren, wie sie im Folgenden genauer beschrieben sind.
Ein Polyorganosiloxan mit endständigen Silangruppen weist den Vorteil auf, dass es im ausgehärteten Zustand besonders wasser- und lichtbeständig ist und besonders weichelastische Eigenschaften ermöglicht.
Ein Silangruppen-haltiges organisches Polymer weist den Vorteil auf, dass es besonders gute Haftungseigenschaften auf einer Vielzahl von Untergründen aufweist und besonders kostengünstig ist.

Bevorzugt wird das Silan der Formel (I) für diese Verwendung getrennt von der härtbaren Zusammensetzung hergestellt, also nicht in-situ in der Zusammensetzung aus einem Hydroxy-Amidin oder -Guanidin der Formel (III) erzeugt. Dabei wird der bei der Umesterung aus dem Alkoxy- oder Ketoximato-Silan freigesetzte Alkohol bzw. das freigesetzte Ketoxim bevorzugt entfernt, bevor das Silan der Formel (I) mit der härtbaren Zusammensetzung kontaktiert wird. Eine solche Zusammensetzung weist den Vorteil auf, dass sie bei Kontakt mit Feuchtigkeit besonders geringe Mengen an flüchtigen organischen Verbindungen freisetzt. Weiterhin weist dieses Vorgehen den Vorteil auf, dass das Silan der Formel (I) in gewissen Zusammensetzungen, insbesondere solchen niedriger Polarität, besser verträglich ist als das zugrunde liegende Hydroxy-Amidin oder -Guanidin der Formel (III). Weiterhin kann das Silan der Formel (I) vor dem Kontaktieren mit der Zusammensetzung mit einem Siliconöl zu einem Umsetzungsprodukt kondensiert werden, was inbesondere im Fall von Zusammensetzungen enthaltend Polyorganosiloxane mit endständigen Silangruppen aufgrund einer noch besseren Verträglichkeit vorteilhaft sein kann.

Ein weiterer Gegenstand der Erfindung ist somit eine Zusammensetzung umfassend mindestens ein Silan der Formel (I) und/oder mindestens ein Amidin- oder Guanidingruppen-haltiges Umsetzungsprodukt davon und mindestens ein Silangruppen-haltiges Polymer.

Eine solche Zusammensetzung verfügt typischerweise über eine gute Lagerfähigkeit ohne Separationsneigung, erlaubt aufgrund der geringen Toxizität und geringen Flüchtigkeit des Silans der Formel (I) eine niedrige Gefahreneinstufung und ermöglicht emissions- und geruchsarme Produkte, welche rasch aushärten und dabei ein mechanisch hochwertiges und beständiges Material bilden. Besonders vorteilhaft ist dabei der Umstand, dass dieses Material kaum zu migrationsbedingten Fehlern wie Ausschwitzen oder Substratverschmutzung neigt, im Gegensatz zu Zusammensetzungen enthaltend Katalysatoren nach dem Stand der Technik, wie beispielsweise DBU oder TMG. Zusammensetzungen enthaltend solche aus dem Stand der Technik bekannten Katalysatoren neigen zu Migrationseffekten, was sich vor der Aushärtung durch Separation und nach der Aushärtung durch klebrige und/oder schmierige Oberflächen und/oder Substratverschmutzungen äussern kann. Besonders letztere Effekte sind äusserst unerwünscht, da klebrige und schmierige Oberflächen schnell verschmutzen und schlecht überstreichbar sind, und Substratverunreinigungen zu bleibenden Verfärbungen führen können.

Das Silangruppen-haltige Polymer ist in einer bevorzugten Ausführungsform ein Polyorganosiloxan mit endständigen Silangruppen.
Ein bevorzugtes Polyorganosiloxan mit endständigen Silangruppen weist die Formel (V) auf, 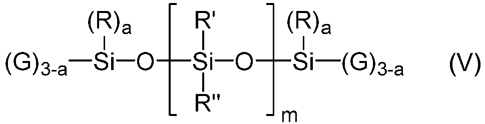 wobei
R, R' und R" unabhängig voneinander jeweils für einen einwertigen Kohlenwasserstoff-Rest mit 1 bis 12 C-Atomen stehen;
G für einen Hydroxyl-Rest oder für einen Alkoxy-, Acetoxy-, Ketoximato-, Amido- oder Enoxy-Rest mit 1 bis 13 C-Atomen steht;
a für 0, 1 oder 2 steht; und
m für eine ganze Zahl im Bereich von 50 bis etwa 2'500 steht.

R steht bevorzugt für Methyl, Vinyl oder Phenyl.
R' und R" stehen bevorzugt unabhängig voneinander jeweils für einen Alkylrest mit 1 bis 5, bevorzugt 1 bis 3 C-Atomen, insbesondere für Methyl.
G steht bevorzugt für einen Hydroxyl-Rest oder für einen Alkoxy- oder Ketoximato-Rest mit 1 bis 6 C-Atomen, insbesondere für einen Hydroxyl-, Methoxy-, Ethoxy-, Methylethylketoximato- oder Methylisobutylketoximato-Rest. Besonders bevorzugt steht G für einen Hydroxylrest.
a steht bevorzugt für 0 oder 1, insbesondere für 0.
Weiterhin ist m bevorzugt so gewählt, dass das Polyorganosiloxan der Formel (V) bei Raumtemperatur eine Viskosität im Bereich von 100 bis 500'000 mPa·s, insbesondere von 1000 bis 100'000 mPa·s, aufweist.

Polyorganosiloxane der Formel (V) sind gut handhabbar und vernetzen mit Feuchtigkeit und/oder Silanvernetzern zu festen Silicon-Polymeren mit elastischen Eigenschaften.
Geeignete kommerziell erhältliche Polyorganosiloxane der Formel (V) sind beispielsweise erhältlich von Wacker, Momentive Performance Material, GE Advanced Materials, Dow Corning, Bayer oder Shin Etsu.

Bevorzugt enthält die Zusammensetzung zusätzlich zum Polyorganosiloxan mit endständigen Silangruppen einen Silanvernetzer, insbesondere einen Silanvernetzer der Formel (VI),

(R'")_{q}-Si-(G')_{4-q} (VI)

wobei
R'" für einen einwertigen Kohlenwasserstoff-Rest mit 1 bis 12 C-Atomen steht, G' für einen Hydroxyl-Rest oder für einen Alkoxy-, Acetoxy-, Ketoximato-, Amido- oder Enoxy-Rest mit 1 bis 13 C-Atomen steht; und
q für einen Wert von 0, 1 oder 2, insbesondere für 0 oder 1, steht.

Besonders geeignete Silanvernetzer der Formel (VI) sind Methyltrimethoxysilan, Ethyltrimethoxysilan, Propytrimethoxysilan, Vinyltrimethoxysilan, Methyltriethoxysilan, Vinyltriethoxysilan, Phenyltriethoxysilan, Tetramethoxysilan, Tetraethoxysilan, Methyltris(methylethylketoximato)silan, Vinyltris(methylethylketoximato)silan und Methyltris(isobutylketoximato)silan.

Das Silangruppen-haltige Polymer ist in einer weiteren bevorzugten Ausführungsform ein Silangruppen-haltiges organisches Polymer, insbesondere ein Polyolefin, Polyester, Polyamid, Poly(meth)acrylat oder Polyether oder eine Mischform dieser Polymere, welches jeweils eine oder bevorzugt mehrere Silangruppen trägt. Die Silangruppen können seitlich in der Kette oder endständig stehen und sind über ein C-Atom an das organische Polymer gebunden. Besonders bevorzugt ist das Silangruppen-haltige organische Polymer ein Silangruppen-haltiges Polyolefin oder ein Silangruppen-haltiger Polyester oder ein Silangruppen-haltiges Poly(meth)acrylat oder ein Silangruppen-haltiger Polyether oder eine Mischform dieser Polymere.
Am meisten bevorzugt ist das Silangruppen-haltige organische Polymer ein Silangruppen-haltiger Polyether.

Das Silangruppen-haltige organische Polymer weist als Silangruppen bevorzugt Alkoxysilangruppen auf, insbesondere Alkoxysilangruppen der Formel (VII), 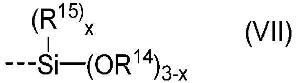 wobei
R¹⁴ für einen linearen oder verzweigten, einwertigen Kohlenwasserstoffrest mit 1 bis 5 C-Atomen, insbesondere für Methyl oder für Ethyl oder für Isopropyl, steht;
R¹⁵ für einen linearen oder verzweigten, einwertigen Kohlenwasserstoffrest mit 1 bis 8 C-Atomen, insbesondere für Methyl oder für Ethyl, steht; und x für einen Wert von 0 oder 1 oder 2, bevorzugt für 0 oder 1, insbesondere für 0, steht.
Besonders bevorzugt steht R¹⁴ für Methyl oder für Ethyl.
Für bestimmte Anwendungen steht der Rest R¹⁴ bevorzugt für eine Ethylgruppe, da in diesem Fall bei der Aushärtung der Zusammensetzung ökologisch und toxikologisch harmloses Ethanol freigesetzt wird.

Besonders bevorzugt sind Trimethoxysilangruppen, Dimethoxymethylsilangruppen oder Triethoxysilangruppen.
Dabei weisen Methoxysilangruppen den Vorteil auf, dass sie besonders reaktiv sind, und Ethoxysilangruppen weisen den Vorteil auf, dass sie toxikologisch vorteilhaft und besonders lagerstabil sind.

Das Silangruppen-haltige organische Polymer weist im Mittel bevorzugt 1.3 bis 4, insbesondere 1.5 bis 3, besonders bevorzugt 1.7 bis 2.8 Silangruppen pro Molekül auf. Die Silangruppen sind bevorzugt endständig.
Das Silangruppen-haltige organische Polymer weist bevorzugt ein mittleres Molekulargewicht, bestimmt mittels GPC gegenüber Polystyrol-Standard, im Bereich von 1'000 bis 30'000 g/mol, insbesondere von 2'000 bis 20'000 g/mol, auf. Das Silangruppen-haltige organische Polymer weist bevorzugt ein Silan-Equivalentgewicht von 300 bis 25'000 g/Eq, insbesondere von 500 bis 15'000 g/Eq, auf.

Das Silangruppen-haltige organische Polymer kann bei Raumtemperatur fest oder flüssig vorliegen. Bevorzugt ist es bei Raumtemperatur flüssig.

Meist bevorzugt handelt es sich beim Silangruppen-haltigen organischen Polymer um einen bei Raumtemperatur flüssigen Silangruppen-haltigen Polyether, wobei die Silangruppen insbesondere Dialkoxysilangruppen und/oder Trialkoxysilangruppen, besonders bevorzugt Trimethoxysilangruppen oder Triethoxysilangruppen, sind.

Verfahren zur Herstellung von Silangruppen-haltigen Polyethern sind dem Fachmann bekannt.
In einem bevorzugten Verfahren sind Silangruppen-haltige Polyether erhältlich aus der Umsetzung von Allylgruppen-haltigen Polyethern mit Hydrosilanen, gegebenenfalls unter Kettenverlängerung mit beispielsweise Diisocyanaten. In einem weiteren bevorzugten Verfahren sind Silangruppen-haltige Polyether erhältlich aus der Copolymerisation von Alkylenoxiden und Epoxysilanen, gegebenenfalls unter Kettenverlängerung mit beispielsweise Diisocyanaten.

In einem weiteren bevorzugten Verfahren sind Silangruppen-haltige Polyether erhältlich aus der Umsetzung von Polyetherpolyolen mit Isocyanatosilanen, gegebenenfalls unter Kettenverlängerung mit Diisocyanaten.
In einem weiteren bevorzugten Verfahren sind Silangruppen-haltige Polyether erhältlich aus der Umsetzung von Isocyanatgruppen-haltigen Polyethern, insbesondere NCO-terminierten Urethan-Polyethern aus der Umsetzung von Polyetherpolyolen mit einer überstöchiometischen Menge an Polyisocyanaten, mit Aminosilanen, Hydroxysilanen oder Mercaptosilanen. Silangruppen-haltige Polyether aus diesem Verfahren sind besonders bevorzugt. Dieses Verfahren ermöglicht den Einsatz einer Vielzahl von kommerziell gut verfügbaren, kostengünstigen Ausgangsmaterialien, womit unterschiedliche Polymereigenschaften erhalten werden können, beispielsweise eine hohe Dehnbarkeit, eine hohe Festigkeit, ein niedriges Elastizitätsmodul, ein tiefer Glasübergangspunkt oder eine hohe Witterungsbeständigkeit.

Besonders bevorzugt ist der Silangruppen-haltige Polyether erhältlich aus der Umsetzung von NCO-terminierten Urethan-Polyethern mit Aminosilanen oder Hydroxysilanen. Geeignete NCO-terminierte Urethan-Polyether sind erhältlich aus der Umsetzung von Polyetherpolyolen, insbesondere Polyoxyalkylendiolen oder Polyoxyalkylentriolen, bevorzugt Polyoxypropylendiolen oder Polyoxypropylentriolen, mit einer überstöchiometrischen Menge an Polyisocyanaten, insbesondere Diisocyanaten.
Bevorzugt wird die Umsetzung zwischen dem Polyisocyanat und dem Polyetherpolyol unter Feuchtigkeitsausschluss bei einer Temperatur von 50 °C bis 160 °C durchgeführt, gegebenenfalls in Anwesenheit geeigneter Katalysatoren, wobei das Polyisocyanat so dosiert ist, dass dessen Isocyanatgruppen im Verhältnis zu den Hydroxylgruppen des Polyols im stöchiometrischen Überschuss vorhanden sind. Insbesondere wird der Überschuss an Polyisocyanat so gewählt, dass im resultierenden Urethan-Polyether nach der Umsetzung aller Hydroxylgruppen ein Gehalt an freien Isocyanatgruppen von 0.1 bis 5 Gewichts-%, bevorzugt 0.2 bis 4 Gewichts-%, besonders bevorzugt 0.3 bis 3 Gewichts-%, bezogen auf das gesamte Polymer, verbleibt.

Bevorzugte Diisocyanate sind ausgewählt aus der Gruppe bestehend aus 1,6-Hexamethylendiisocyanat (HDI), 1-Isocyanato-3,3,5-trimethyl-5-isocyanatomethyl-cyclohexan (=Isophorondiisocyanat oder IPDI), 2,4- und 2,6-Toluylendiisocyanat und beliebige Gemische dieser Isomeren (TDI) und 4,4'-, 2,4'- und 2,2'-Diphenylmethandiisocyanat und beliebige Gemische dieser Isomeren (MDI). Besonders bevorzugt sind IPDI oder TDI. Meist bevorzugt ist IPDI. Damit werden Silangruppen-haltige Polyether mit besonders guter Lichtechtheit erhalten.

Speziell geeignet als Polyetherpolyole sind Polyoxyalkylendiole oder Polyoxyalkylentriole mit einem Ungesättigtheitsgrad tiefer als 0.02 mEq/g, insbesondere tiefer als 0.01 mEq/g, und einem mittleren Molekulargewicht im Bereich von 400 bis 25'000 g/mol, insbesondere 1000 bis 20'000 g/mol.
Neben Polyetherpolyolen können anteilig auch andere Polyole eingesetzt werden, insbesondere Polyacrylatpolyole, sowie niedrigmolekulare Diole oder Triole.

Geeignete Aminosilane für die Umsetzung mit einem NCO-terminierten Urethan-Polyether sind primäre und sekundäre Aminosilane. Bevorzugt sind 3-Aminopropyltrimethoxysilan, 3-Aminopropyldimethoxymethylsilan, 4-Aminobutyl-trimethoxysilan, 4-Amino-3-methylbutyl-trimethoxysilan, 4-Amino-3,3-dimethylbutyl-trimethoxysilan, N-Butyl-3-aminopropyltrimethoxysilan, N-Phenyl-3-aminopropyltrimethoxysilan, Addukte aus primären Aminosilanen wie 3-Aminopropyltrimethoxysilan, 3-Aminopropyldimethoxymethylsilan oder N-(2-Aminoethyl)-3-aminopropyltrimethoxysilan und Michael-Akzeptoren wie Acrylnitril, (Meth)acrylsäureestern, (Meth)acrylsäureamiden, Maleinsäure- oder Fumarsäurediestern, Citraconsäurediestern oder Itaconsäurediestern, insbesondere N-(3-Trimethoxysilylpropyl)amino-bernsteinsäuredimethyl- oder -diethylester. Ebenfalls geeignet sind Analoga der genannten Aminosilane mit Ethoxy- oder Isopropoxygruppen anstelle der Methoxygruppen am Silicium.

Geeignete Hydroxysilane für die Umsetzung mit einem NCO-terminierten Urethan-Polyether sind insbesondere erhältlich aus der Addition von Aminosilanen an Lactone oder an cyclische Carbonate oder an Lactide.

Dafür geeignete Aminosilane sind insbesondere 3-Aminopropyl-trimethoxysilan, 3-Aminopropyl-triethoxysilan, 4-Aminobutyl-trimethoxysilan, 4-Aminobutyl-triethoxysilan, 4-Amino-3-methylbutyl-trimethoxysilan, 4-Amino-3-methylbutyl-triethoxysilan, 4-Amino-3,3-dimethylbutyl-trimethoxysilan, 4-Amino-3,3-dimethylbutyl-triethoxysilan, 2-Aminoethyl-trimethoxysilan oder 2-Aminoethyltriethoxysilan. Besonders bevorzugt sind 3-Aminopropyl-trimethoxysilan, 3-Aminopropyl-triethoxysilan, 4-Amino-3,3-dimethylbutyl-trimethoxysilan oder 4-Amino-3,3-dimethylbutyl-triethoxysilan.
Als Lactone geeignet sind insbesondere γ-Valerolacton, γ-Octalacton, δ-Decalacton, und ε-Decalacton, insbesondere γ-Valerolacton.
Als cyclische Carbonate eignen sich insbesondere 4,5-Dimethyl-1,3-dioxolan-2-on, 4,4-Dimethyl-1,3-dioxolan-2-on, 4-Ethyl-1,3-dioxolan-2-on, 4-Methyl-1,3-dioxolan-2-on oder 4-(Phenoxymethyl)-1,3-dioxolan-2-on.
Als Lactide eignen sich insbesondere 1,4-Dioxan-2,5-dion (Lactid aus 2-Hydroxyessigsäure, auch "Glycolid" genannt), 3,6-Dimethyl-1,4-dioxan-2,5-dion (Lactid aus Milchsäure, auch "Lactid" genannt) und 3,6-Diphenyl-1,4-dioxan-2,5-dion (Lactid aus Mandelsäure).
Bevorzugte Hydroxysilane, welche auf diese Weise erhalten werden, sind N-(3-Triethoxysilylpropyl)-2-hydroxypropanamid, N-(3-Trimethoxysilylpropyl)-2-hydroxypropanamid, N-(3-Triethoxysilylpropyl)-4-hydroxypentanamid, N-(3-Tri-ethoxysilylpropyl)-4-hydroxyoctanamid, N-(3-Triethoxysilylpropyl)-5-hydroxy-decanamid und N-(3-Triethoxysilylpropyl)-2-hydroxypropylcarbamat.

Weiterhin sind geeignete Hydroxysilane auch erhältlich aus der Addition von Aminosilanen an Epoxide oder aus der Addition von Aminen an Epoxysilane. Bevorzugte Hydroxysilane, welche auf diese Weise erhalten werden, sind 2-Morpholino-4(5)-(2-trimethoxysilylethyl)cyclohexan-1-ol, 2-Morpholino-4(5)-(2-triethoxysilylethyl)cyclohexan-1-ol oder 1-Morpholino-3-(3-(triethoxysilyl)pro-poxy)propan-2-ol.

Als Silangruppen-haltige Polyether sind auch kommerziell erhältliche Produkte geeignet, insbesondere die Folgenden: MS Polymer™ (von Kaneka Corp.; insbesondere die Typen S203H, S303H, S227, S810, MA903 und S943); MS Polymer™ bzw. Silyl™ (von Kaneka Corp.; insbesondere die Typen SAT010, SAT030, SAT200, SAX350, SAX400, SAX725, MAX450, MAX951); Excestar® (von Asahi Glass Co. Ltd.; insbesondere die Typen S2410, S2420, S3430, S3630); SPUR+* (von Momentive Performance Materials; insbesondere die Typen 1010LM, 1015LM, 1050MM); Vorasil™ (von Dow Chemical Co.; insbesondere die Typen 602 und 604); Desmoseal® (von Bayer MaterialScience AG; insbesondere die Typen S XP 2458, S XP 2636, S XP 2749, S XP 2774 und S XP 2821), TEGOPAC® (von Evonik Industries AG; insbesondere die Typen Seal 100, Bond 150, Bond 250), Polymer ST (von Hanse Chemie AG/Evonik Industries AG, insbesondere die Typen 47, 48, 61, 61LV, 77, 80, 81); Geniosil® STP (von Wacker Chemie AG; insbesondere die Typen E10, E15, E30, E35).

Besonders bevorzugte Silangruppen-haltigen organische Polymere weisen Endgruppen der Formel (VIII) auf, 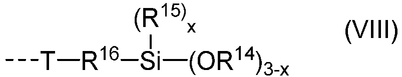 wobei
R¹⁶ für einen linearen oder verzweigten, zweiwertigen Kohlenwasserstoffrest mit 1 bis 12 C-Atomen, welcher gegebenenfalls cyclische und/oder aromatische Anteile und gegebenenfalls ein oder mehrere Heteroatome, insbesondere ein oder mehrere Stickstoffatome, aufweist, steht;
T für einen zweiwertigen Rest ausgewählt aus -O-, -S-, -N(R¹⁷)-, -O-CO-N(R¹⁷)-, -N(R¹⁷)-CO-O- und -N(R¹⁷)-CO-N(R¹⁷)- steht,
   wobei R¹⁷ für einen Wasserstoff-Rest oder für einen linearen oder verzweigten Kohlenwasserstoff-Rest mit 1 bis 20 C-Atomen, welcher gegebenenfalls cyclische Anteile aufweist, und welcher gegebenenfalls eine Alkoxysilan-, Ether- oder Carbonsäureestergruppe aufweist, steht; und
R¹⁴, R¹⁵ und x die bereits genannten Bedeutungen aufweisen.
Bevorzugt steht R¹⁶ für 1,3-Propylen oder für 1,4-Butylen, wobei Butylen mit einer oder zwei Methylgruppen substituiert sein kann.
Besonders bevorzugt steht R¹⁶ für 1,3-Propylen.

Bevorzugt ist das Silan der Formel (I) in der Zusammensetzung in einer solchen Menge vorhanden, dass die Konzentration an Amidin- oder Guanidin-Gruppen aus dem Silan der Formel (I) bezogen auf die Menge des vernetzbaren Polymers im Bereich von 0.1 bis 20 mmol/100 g Polymer, bevorzugt 0.1 bis 15 mmol/100 g Polymer, insbesondere 0.1 bis 10 mmol/100 g, liegt. Eine solche Zusammensetzung weist eine gute Lagerfähigkeit und eine schnelle Aushärtung auf.

Zusätzlich zum beschriebenen Silan der Formel (I) und/oder einem Amidin- oder Guanidingruppen-haltigen Umsetzungsprodukt davon kann die Zusammensetzung weitere Katalysatoren, insbesondere für die Vernetzung von Silangruppen, enthalten. Als weitere Katalysatoren geeignet sind insbesondere Metall-Verbindungen und/oder basische Stickstoff- oder Phosphorverbindungen.

Geeignete Metall-Verbindungen sind insbesondere Verbindungen von Zinn, Titan, Zirkonium, Aluminium oder Zink, insbesondere Diorganozinn(IV)-Verbindungen wie insbesondere Dibutylzinn(IV)-diacetat, Dibutylzinn(IV)-dilaurat, Dibutylzinn(IV)-dineodecanoat oder Dibutylzinn(IV)-bis(acetylacetonat) und Dioctylzinn(IV)-dilaurat, sowie Titan(IV)- oder Zirkonium(IV)- oder Aluminium(III)- oder Zink(II)-Komplexe mit insbesondere Alkoxy-, Carboxylat-, 1,3-Diketonat-, 1,3-Ketoesterat- oder 1,3-Ketoamidat-Liganden.

Geeignete basische Stickstoff- oder Phosphorverbindungen sind insbesondere Imidazole, Pyridine, Phosphazen-Basen oder bevorzugt Amine, Hexahydrotriazine, Biguanide, sowie weitere Amidine oder Guanidine.

Geeignete Amine sind insbesondere Alkyl-, Cycloalkyl- oder Aralkylamine wie Triethylamin, Triisopropylamin, 1-Butylamin, 2-Butylamin, tert.Butylamin, 3-Methyl-1-butylamin, 3-Methyl-2-butylamin, Dibutylamin, Tributylamin, Hexylamin, Dihexylamin, Cyclohexylamin, Dicyclohexylamin, Dimethylcyclohexylamin, Benzylamin, Dibenzylamin, Dimethylbenzylamin, Octylamin, 2-Ethylhexylamin, Di-(2-ethylhexyl)amin, Laurylamin, N,N-Dimethyl-laurylamin, Stearylamin, N,N-Dimethyl-stearylamin; von natürlichen Fettsäuregemischen abgeleitete Fettamine, wie insbesondere Cocoalkylamin, N,N-Dimethyl-cocoalkylamin, C₁₆₋₂₂-Alkylamin, N,N-Dimethyl-C₁₆₋₂₂-alkylamin, Soyaalkylamin, N,N-Dimethyl-soyaalkylamin, Oleylamin, N,N-Dimethyl-oleylamin, Talgalkylamin oder N,N-Dimethyl-talgalkylamin, erhältlich beispielsweise unter den Handelsnamen Armeen® (von Akzo Nobel) oder Rofamin® (von Ecogreen Oleochemicals); aliphatische, cycloaliphatische oder araliphatische Diamine wie Ethylendiamin, Butandiamin, Hexamethylendiamin, Dodecandiamin, Neopentandiamin, 2-Methyl-pentamethylendiamin (MPMD), 2,2(4),4-Trimethylhexamethylendiamin (TMD), Isophorondiamin (IPD), 2,5(2,6)-Bis-(aminomethyl)-bicy-clo[2.2.1]heptan (NBDA), 1,3-Xylylendiamin (MXDA), N,N'-Di(tert.butyl)ethylendiamin, N,N,N',N'-Tetramethyl-ethylendiamin, N,N,N',N'-Tetramethyl-propylendiamin, N,N,N',N'-Tetramethyl-hexamethylendiamin, 3-Dimethylaminopropylamin, 3-(Methylamino)propylamin, 3-(Cyclohexylamino)propylamin, Piperazin, N-Methylpiperazin, N,N'-Dimethylpiperazin, 1,4-Diazabicyclo[2.2.2]octan, Fettpolyamine wie N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Alkyl)-1,3-propandiamin, erhältlich beispielsweise unter dem Handelsnamen Duomeen® (von Akzo Nobel); Polyalkylenamine wie Diethylentriamin, Dipropylentriamin, Triethylentetramin (TETA), Tetraethylenpentamin (TEPA), Pentamethylenhexamin (PEHA), 3-(2-Aminoethyl)aminopropylamin, N,N'-Bis(3-aminopropyl)ethylendiamin, N-(3-Aminopropyl)-N-methylpropandiamin, Bis(3-dimethylaminopropyl)amin, N-(3-Dimethylaminopropyl)-1,3-propylendiamin, N-(2-Aminoethyl)piperazin (N-AEP), N-(2-Aminopropyl)piperazin, N,N'-Di-(2-aminoethyl)piperazin, 1-Methyl-4-(2-dimethylaminoethyl)piperazin, N,N,N',N",N"-Pentamethyldiethylentriamin, N,N,N',N",N"-Pentamethyldipropylentriamin, Polyethylenimine, erhältlich beispielsweise unter den Handelsnamen Lupasol® (von BASF) und Epomin® (von Nippon Shokubai); Etheramine, wie insbesondere 2-Methoxyethylamin, 2-Ethoxyethylamin, 3-Methoxypropylamin, 3-Ethoxypropylamin, 3-(2-Ethylhexyloxy)propylamin, 3-(2-Methoxyethoxy)propylamin, 2(4)-Methoxyphenylethylamin, Morpholin, N-Methylmorpholin, N-Ethylmorpholin, 2-Aminoethylmorpholin, Bis(2-aminoethyl)ether, Bis(dimethylaminoethyl)-ether, Bis(dimorpholinoethyl)ether, N,N,N'-Trimethyl-N'-hydroxyethylbis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin oder 2-Aminopropyl-terminierte Glykole, wie sie beispielsweise unter dem Handelsnamen Jeffamin® (von Huntsman) erhältlich sind; Aminoalkohole, wie insbesondere Ethanolamin, Isopropanolamin, Diethanolamin, Diisopopanolamin, Triethanolamin, Triisopropanolamin, N-Butylethanolamin, Diglykolamin, N,N-Diethylethanolamin, N-Methyldiethanolamin, N-Methyldiisopropylamin, N,N,N'-Trimethylaminoethyl-ethanolamin, N-(3-Dimethylaminopropyl)-N,N-diisopropanolamin, N,N-Bis(3-dimethylaminopropyl)-N-isopropanolamin, 2-(2-Dimethylaminoethoxy)ethanolamin oder Addukte aus Mono- und Polyaminen mit Epoxiden oder Diepoxiden; Phenolgruppen-haltige Amine, wie insbesondere Kondensationsprodukte aus Phenolen, Aldehyden und Aminen (sogenannte Mannich-Basen und Phenalkamine) wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin sowie im Handel unter den Markennamen Cardolite® (von Cardolite), Aradur® (von Huntsman) und Beckopox® (von Cytec) erhältliche Phenalkamine; Amidgruppen-haltige Polyamine, sogenannte Polyamidoamine, wie sie im Handel erhältlich sind, beispielsweise unter den Markennamen Versamid® (von Cognis), Aradur® (von Huntsman), Euretek® (von Huntsman) oder Beckopox® (von Cytec); oder Aminosilane, wie insbesondere 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropyl-methyldimethoxysilan, N-(2-Aminoethyl)-N'-[3-(trimethoxysilyl)propyl]ethylendiamin oder deren Analoga mit Ethoxy- anstelle der Methoxygruppen am Silicium.
Geeignete Hexahydrotriazine sind insbesondere 1,3,5-Hexahydrotriazin oder 1,3,5-Tris(3-(dimethylamino)propyl)-hexahydrotriazin.
Geeignete Biguanide sind insbesondere Biguanid, 1-Butylbiguanid, 1,1-Dimethylbiguanid, 1-Butylbiguanid, 1-Phenylbiguanid oder 1-(o-Tolyl)biguanid (OTBG).
Geeignete weitere Amidine sind insbesondere 1,8-Diazabicyclo[5.4.0]undec-7-en (DBU), 1,5-Diazabicyclo[4.3.0]non-5-en (DBN), 6-Dibutylamino-1,8-diazabicyclo[5.4.0]undec-7-en, 6-Dibutylamino-1,8-diazabicyclo[5.4.0]undec-7-en, N,N'-Di-n-hexylacetamidin (DHA), 2-Methyl-1,4,5,6-tetrahydropyrimidin, 1,2-Dimethyl-1,4,5,6-tetrahydropyrimidin, 2,5,5-Trimethyl-1,4,5,6-tetrahydropyrimidin, N-(3-Trimethoxysilylpropyl)-4,5-dihydroimidazol oder N-(3-Triethoxysilylpropyl)-4,5-dihydroimidazol.
Geeignete weitere Guanidine sind insbesondere 1-Butylguanidin, 1,1-Dimethylguanidin, 1,3-Dimethylguanidin, 1,1,3,3-Tetramethylguanidin (TMG), 2-(3-(Trimethoxysilyl)propyl)-1,1,3,3-tetramethylguanidin, 2-(3-(Methyldimethoxysilyl)propyl)-1,1,3,3-tetramethylguanidin, 2-(3-(Triethoxysilyl)propyl)-1,1,3,3-tetramethylguanidin, 1,5,7-Triazabicyclo[4.4.0]dec-5-en (TBD), 7-Methyl-1,5,7-triazabicyclo[4.4.0]dec-5-en, 7-Cyclohexyl-1,5,7-triazabicyclo[4.4.0]dec-5-en, 1-Phenylguanidin, 1-(o-Tolyl)guanidin (OTG), 1,3-Diphenylguanidin, 1,3-Di(o-tolyl)guanidin oder 2-Guanidinobenzimidazol.

Weiterhin kann die Zusammensetzung als Co-Katalysator eine Säure enthalten, insbesondere eine Carbonsäure. Bevorzugt sind aliphatische Carbonsäuren wie Ameisensäure, Laurinsäure, Stearinsäure, Isostearinsäure, Ölsäure, 2-Ethyl-2,5-dimethylcapronsäure, 2-Ethylhexansäure, Neodecansäure, Fettsäuregemische aus der Verseifung von natürlichen Fetten und Ölen oder Di- und Polycarbonsäuren, insbesondere Poly(meth)acrylsäuren.

Die Zusammensetzung ist in einer bevorzugten Ausführungsform im Wesentlichen frei von Organozinn-Verbindungen. Organozinn-freie Zusammensetzungen sind hinsichtlich Gesundheitsschutz und Umweltschutz vorteilhaft. Insbesondere beträgt der Zinngehalt der härtbaren Zusammensetzung weniger als 0.1 Gew.-%, insbesondere weniger als 0.05 Gew.-%.

Die Zusammensetzung enthält in einer weiteren bevorzugten Ausführungsform eine Kombination aus mindestens Silan der Formel (I) und/oder einem Amidin- oder Guanidingruppen-haltigen Umsetzungsprodukt davon und mindestens einer Organozinn-Verbindung, insbesondere einer Diorganozinn(IV)-Verbindung wie die vorgängig genannten. Eine solche Zusammensetzung weist bereits bei einem geringen Zinn-Gehalt eine hohe Aushärtungsgeschwindigkeit auf, was aus toxikologischen und ökologischen Gründen vorteilhaft ist.

In einer Ausführungsform der Erfindung enthält die Zusammensetzung mindestens ein Organotitanat. Eine Kombination aus einem Silan der Formel (I) und/oder einem Amidin- oder Guanidingruppen-haltigen Umsetzungsprodukt davon und einem Organotitanat weist eine besonders hohe katalytische Aktivität auf. Dadurch wird eine schnelle Aushärtung einer solchen Zusammensetzung bei einer verhältnismässig geringen Einsatzmenge an Organotitanat ermöglicht.
Als Organotitanat geeignet sind insbesondere Titan(IV)-Komplexverbindungen. Bevorzugte Organoititanate sind insbesondere ausgewählt aus
- Titan(IV)-Komplexverbindungen mit zwei 1,3-Diketonat-Liganden, insbesondere 2,4-Pentandionat (=Acetylacetonat), und zwei Alkoholat-Liganden;
- Titan(IV)-Komplexverbindungen mit zwei 1,3-Ketoesterat-Liganden, insbesondere Ethylacetoacetat, und zwei Alkoholat-Liganden;
- Titan(IV)-Komplexverbindungen mit einem oder mehreren Aminoalkoholat-Liganden, insbesondere Triethanolamin oder 2-((2-Aminoethyl)amino)ethanol, und einem oder mehreren Alkoholat-Liganden;
- Titan(IV)-Komplexverbindungen mit vier Alkoholat-Liganden;
- sowie höherkondensierte Organotitanate, insbesondere oligomeres Titan(IV)-tetrabutanolat, auch als Polybutyltitanat bezeichnet;
wobei als Alkoholat-Liganden insbesondere Isobutoxy, n-Butoxy, Isopropoxy, Ethoxy und 2-Ethylhexoxy geeignet sind.

Insbesondere geeignet sind die kommerziell erhältlichen Typen Tyzor® AA, GBA, GBO, AA-75, AA-65, AA-105, DC, BEAT, BTP, TE, TnBT, KTM, TOT, TPT oder IBAY (alle von Dorf Ketal); Tytan PBT, TET, X85, TAA, ET, S2, S4 oder S6 (alle von Borica Company Ltd.) und Ken-React® KR® TTS, 7, 9QS, 12, 26S, 33DS, 38S, 39DS, 44, 134S, 138S, 133DS, 158FS oder LICA® 44 (alle von Kenrich Petrochemicals).

Ganz besonders geeignete Organotitanate sind ausgewählt aus Bis(ethylacetoacetato)diisobutoxy-titan(IV) (kommerziell erhältlich beispielsweise als Tyzor® IBAY von Dorf Ketal), Bis(ethylacetoacetato)diisopropoxy-titan(IV) (kommerziell erhältlich beispielsweise als Tyzor® DC von Dorf Ketal), Bis(acetylacetonato)diisopropoxy-titan(IV), Bis(acetylacetonato)diisobutoxy-titan(IV), Tris(oxyethyl)-amin-isopropoxy-titan(IV), Bis[tris(oxyethyl)amin]diisopropoxy-titan(IV), Bis(2-ethylhexan-1,3-dioxy)-titan(IV), Tris[2-((2-Aminoethyl)amino)ethoxy]ethoxy-titan(IV), Bis(neopentyl(diallyl)oxy)-diethoxy-titan(IV), Titan(IV)-tetrabutanolat, Tetra(2-ethylhexyloxy)titanat, Tetra(isopropoxy)titanat und Polybutyltitanat. Am meisten bevorzugt sind Bis(ethylacetoacetato)diisobutoxy-titan(IV) oder Bis(ethylacetoacetato)diisopropoxy-titan(IV).

Die Zusammensetzung kann zusätzlich zum Silan der Formel (I) und/oder einem Amidin- oder Guanidingruppen-haltigen Umsetzungsprodukt davon weitere Bestandteile enthalten, insbesondere die folgenden Hilfs- und Zusatzmittel:
- Haftvermittler und/oder Vernetzer, insbesondere Aminosilane wie insbesondere 3-Aminopropyl-trimethoxysilan, 3-Aminopropyl-dimethoxymethylsilan, N-(2-Aminoethyl)-3-aminopropyl-trimethoxysilan, N-(2-Aminoethyl)-3-aminopropyl-dimethoxymethylsilan, N-(2-Aminoethyl)-N'-[3-(trimethoxysilyl)propyl]ethylendiamin oder deren Analoga mit Ethoxy- anstelle von Methoxygruppen, weiterhin N-Phenyl-, N-Cyclohexyl- oder N-Alkylaminosilane, Mercaptosilane, Epoxysilane, (Meth)acrylosilane, Anhydridosilane, Carbamatosilane, Alkylsilane oder Iminosilane, oligomere Formen dieser Silane, Addukte aus primären Aminosilanen mit Epoxysilanen oder (Meth)acrylosilanen oder Anhydridosilanen, aminofunktionelle Alkylsilsesquioxane, insbesondere aminofunktionelles Methylsilsesquioxan oder aminofunktionelles Propylsilsesquioxan. Insbesondere geeignet sind 3-Aminopropyltrimethoxysilan, 3-Aminopropyltriethoxysilan, N-(2-Aminoethyl)-3-aminopropyltrimethoxysilan, N-(2-Aminoethyl)-3-aminopropyl-triethoxysilan, 3-Glycidoxypropyltrimethoxysilan, 3-Glycidoxypropyltriethoxysilan oder 3-Ureidopropyltrimethoxysilan, oder oligomere Formen dieser Silane;
- Trocknungsmittel, insbesondere Tetraethoxysilan, Vinyltrimethoxysilan, Vinyltriethoxysilan oder Organoalkoxysilane, welche in α-Stellung zur Silangruppe eine funktionelle Gruppe aufweisen, insbesondere N-(Methyldimethoxysilylmethyl)-O-methyl-carbamat, (Methacryloxymethyl)silane, Methoxymethylsilane, Orthoameisensäureester, Calciumoxid oder Molekularsiebe, insbesondere Vinyltrimethoxysilan oder Vinyltriethoxysilan;
- Weichmacher, insbesondere Trialkylsilyl-terminierte Polydialkylsiloxane, vorzugsweise Trimethylsilyl-terminierte Polydimethylsiloxane, insbesondere mit Viskositäten im Bereich von 10 bis 1'000 mPa·s, oder entsprechende Verbindungen, bei denen einige der Methylgruppen ersetzt sind durch andere organische Gruppen, insbesondere Phenyl-, Vinyl- oder Trifluorpropylgruppen, sogenannte Reaktivweichmacher in Form von monofunktionellen, also einseitig reaktiven, Polysiloxanen, Carbonsäureester wie Phthalate, insbesondere Dioctylphthalat, Bis(2-ethylhexyl)phthalat, Bis(3-propylheptyl)-phthalat, Diisononylphthalat oder Diisodecylphthalat, Diester von ortho-Cyclohexandicarbonsäure, insbesondere Diisononyl-1,2-cyclohexandicarboxylat, Adipate, insbesondere Dioctyladipat, Bis(2-Ethylhexyl)adipat, Azelate, insbesondere Bis(2-ethylhexyl)acelat, Sebacate, insbesondere Bis(2-ethylhexyl)sebacat oder Diisononylsebacat, Polyole, insbesondere Polyoxyalkylenpolyole oder Polyesterpolyole, Glykolether, Glykolester, organische Phosphor- oder Sulfonsäureester, Sulfonsäureamide, Polybutene oder von natürlichen Fetten oder Ölen abgeleitete Fettsäuremethyl- oder -ethylester, auch "Biodiesel" genannt, wobei Siloxangruppen-haltige Weichmacher besonders geeignet sind für Silangruppen-haltige Polymere in Form von Polyorganosiloxanen;
- Lösemittel;
- anorganische oder organische Füllstoffe, insbesondere natürliche, gemahlene oder gefällte Calciumcarbonate, welche gegebenenfalls mit Fettsäuren, insbesondere Stearinsäure, beschichtet sind, Baryt (Schwerspat), Talke, Quarzmehle, Quarzsand, Dolomite, Wollastonite, Kaoline, calcinierte Kaoline, Mica (Kalium-Aluminium-Silikat), Molekularsiebe, Aluminiumoxide, Aluminiumhydroxide, Magnesiumhydroxid, Kieselsäuren inklusive hochdisperse Kieselsäuren aus Pyrolyseprozessen, industriell hergestellte Russe, Graphit, Metall-Pulver wie Aluminium, Kupfer, Eisen, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Farbstoffe;
- Pigmente, insbesondere Titandioxid oder Eisenoxide;
- Rheologie-Modifizierer, insbesondere Verdickungsmittel, insbesondere Schichtsilikate wie Bentonite, Derivate von Rizinusöl, hydriertes Rizinusöl, Polyamide, Polyurethane, Harnstoffverbindungen, pyrogene Kieselsäuren, Celluloseether oder hydrophob modifizierte Polyoxyethylene;
- Stabilisatoren gegen Oxidation, Wärme, Licht oder UV-Strahlung;
- natürliche Harze, Fette oder Öle wie Kolophonium, Schellack, Leinöl, Rizinusöl oder Sojaöl;
- nicht-reaktive Polymere, wie insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid und Magnesiumhydroxid, oder insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)-phosphat, isopropyliertes Triphenylphosphat, Mono-, Bis- oder Tris(isopropylphenyl)phosphate unterschiedlichen Isopropylierungsgrades, Resorcinolbis(diphenylphosphat), Bisphenol-A-bis(diphenylphosphat) oder Ammoniumpolyphosphate;
- oberflächenaktive Substanzen, insbesondere Netzmittel, Verlaufsmittel, Entlüftungsmittel oder Entschäumer;
- Biozide, insbesondere Algizide, Fungizide oder das Pilzwachstum hemmende Substanzen;
sowie weitere üblicherweise in härtbaren Zusammensetzungen eingesetzte Substanzen. Es kann sinnvoll sein, gewisse Bestandteile vor dem Einmischen in die Zusammensetzung chemisch oder physikalisch zu trocknen.

In einer bevorzugten Ausführungsform enthält die Zusammensetzung mindestens ein Trocknungsmittel und mindestens einen Haftvermittler und/oder Vernetzer.
In einer bevorzugten Ausführungsform enthält die Zusammensetzung keine Phthalate als Weichmacher. Solche Zusammensetzungen sind toxikologisch vorteilhaft und weisen weniger Probleme mit Migrationseffekten auf.

Üblicherweise beträgt der Anteil an Silangruppen-haltigem Polymer in der Zusammensetzung 10 bis 80 Gew.-%, insbesondere 15 bis 60 Gew.-%, bevorzugt 15 bis 50 Gew.-%, bezogen auf das Gesamtgewicht der Zusammensetzung.

Die Zusammensetzung wird vorzugsweise unter Ausschluss von Feuchtigkeit hergestellt und aufbewahrt. Typischerweise ist sie in einer geeigneten Verpackung oder Anordnung, wie insbesondere einer Flasche, einer Büchse, einem Beutel, einem Eimer, einem Fass oder einer Kartusche, unter Ausschluss von Feuchtigkeit lagerstabil.

Die Zusammensetzung kann in Form einer einkomponentigen oder in Form einer mehrkomponentigen, insbesondere zweikomponentigen, Zusammensetzung vorliegen.
Als "einkomponentig" wird im vorliegenden Dokument eine Zusammensetzung bezeichnet, bei welcher alle Bestandteile der Zusammensetzung vermischt im gleichen Gebinde gelagert werden und welche mit Feuchtigkeit härtbar ist. Als "zweikomponentig" wird im vorliegenden Dokument eine Zusammensetzung bezeichnet, bei welcher die Bestandteile der Zusammensetzung in zwei verschiedenen Komponenten vorliegen, welche in voneinander getrennten Gebinden gelagert werden. Erst kurz vor oder während der Applikation der Zusammensetzung werden die beiden Komponenten miteinander vermischt, worauf die vermischte Zusammensetzung aushärtet, gegebenenfalls unter Einwirkung von Feuchtigkeit.

Für den Fall, dass die Zusammensetzung ein Polyorganosiloxan mit endständigen Silangruppen enthält, ist sowohl eine einkomponentige Zusammensetzung, auch als RTV-1 bezeichnet, als auch eine zweikomponentige Zusammensetzung, auch als RTV-2 bezeichnet, bevorzugt. Im Fall einer RTV-2 Zusammensetzung ist das Polyorganosiloxan mit endständigen Silangruppen bevorzugt ein Bestandteil der ersten Komponente und ein Silanvernetzer, insbesondere ein Silanvernetzer der Formel (VI), ist bevorzugt ein Bestandteil der zweiten Komponente. Das Silan der Formel (I) kann dabei in der ersten und/oder in der zweiten Komponente enthalten sein.
Für den Fall, dass die Zusammensetzung ein Silangruppen-haltiges organisches Polymer enthält, ist die Zusammensetzung bevorzugt einkomponentig.

Eine zweite oder gegebenenfalls weitere Komponenten wird bzw. werden mit der ersten Komponente vor oder bei der Applikation vermischt, insbesondere über einen Statikmischer oder über einen dynamischen Mischer.
Die Zusammensetzung wird insbesondere umgebungswarm, bevorzugt in einem Temperaturbereich zwischen 0 °C und 45 °C, insbesondere 5 °C bis 35 °C, appliziert und härtet auch bei diesen Bedingungen aus.
Bei der Applikation beginnt die Vernetzungsreaktion der Silangruppen, gegebenenfalls unter Einfluss von Feuchtigkeit. Vorhandene Silangruppen können mit vorhandenen Silanolgruppen zu Siloxangruppen (Si-O-Si-Gruppen) kondensieren. Vorhandene Silangruppen können bei Kontakt mit Feuchtigkeit auch zu Silanolgruppen (Si-OH-Gruppen) hydrolysieren und durch nachfolgende Kondensationsreaktionen Siloxangruppen (Si-O-Si-Gruppen) bilden. Als Ergebnis dieser Reaktionen härtet die Zusammensetzung schliesslich aus. Das Silan der Formel (I) beschleunigt diese Aushärtung.
Falls für die Aushärtung Wasser benötigt wird, kann dieses entweder aus der Luft stammen (Luftfeuchtigkeit), oder aber die Zusammensetzung kann mit einer Wasser enthaltenden Komponente in Kontakt gebracht werden, zum Beispiel durch Bestreichen, beispielsweise mit einem Abglättmittel, oder durch Besprühen, oder es kann der Zusammensetzung bei der Applikation Wasser oder eine Wasser enthaltende Komponente zugesetzt werden, zum Beispiel in Form einer wasserhaltigen oder Wasser freisetzenden Flüssigkeit oder Paste. Eine Paste ist insbesondere geeignet für den Fall, dass die Zusammensetzung selber in Form einer Paste vorliegt.
Bei einer Aushärtung mittels Luftfeuchtigkeit härtet die Zusammensetzung von aussen nach innen durch, wobei zunächst eine Haut an der Oberfläche der Zusammensetzung gebildet wird. Die sogenannte Hautbildungszeit stellt ein Mass für die Aushärtungsgeschwindigkeit der Zusammensetzung dar. Die Geschwindigkeit der Aushärtung wird dabei im Allgemeinen von verschiedenen Faktoren, wie beispielsweise der Verfügbarkeit von Wasser, der Temperatur usw., bestimmt.

Die Zusammensetzung eignet sich für eine Vielzahl von Anwendungen, insbesondere als Anstrich, Lack oder Primer, als Harz zur Herstellung von Faserverbundwerkstoff (Composite), als Hartschaum, Weichschaum, Formteil, Elastomer, Faser, Folie oder Membran, als Vergussmasse, Dichtstoff, Klebstoff, Belag, Beschichtung oder Anstrich für Bau- und Industrieanwendungen, beispielsweise als Nahtabdichtung, Hohlraumversiegelung, Elektroisolationsmasse, Spachtelmasse, Fugendichtstoff, Schweiss- oder Bördelnahtdichtstoff, Montageklebstoff, Karrosserieklebstoff, Scheibenklebstoff, Sandwichelementklebstoff, Kaschierklebstoff, Laminatklebstoff, Verpackungsklebstoff, Holzklebstoff, Parkettklebstoff, Verankerungsklebstoff, Bodenbelag, Bodenbeschichtung, Balkonbeschichtung, Dachbeschichtung, Betonschutzbeschichtung, Parkhausbeschichtung, Versiegelung, Rohrbeschichtung, Korrosionsschutzbeschichtung, Textilbeschichtung, Dämpfungselement, Dichtungselement oder Spachtelmasse.
Besonders geeignet ist die Zusammensetzung als Klebstoff und/oder Dichtstoff, insbesondere für die Fugenabdichtung und für elastische Klebeverbindungen in Bau- und Industrieanwendungen, sowie als elastische Beschichtung mit rissüberbrückenden Eigenschaften, insbesondere zum Schützen und/oder Abdichten von beispielsweise Dächern, Böden, Balkonen, Parkdecks oder Betonröhren.

Bevorzugt stellt die Zusammensetzung somit einen Klebstoff oder einen Dichtstoff oder eine Beschichtung dar.

Eine solche Zusammensetzung enthält typischerweise Weichmacher, Füllstoffe, Haftvermittler und/oder Vernetzer und Trocknungsmittel und gegebenenfalls weitere Hilfs- und Zusatzstoffe.
Für eine Anwendung als Klebstoff oder Dichtstoff weist die Zusammensetzung bevorzugt eine pastöse Konsistenz mit strukturviskosen Eigenschaften auf. Ein solcher pastöser Dichtstoff oder Klebstoff wird insbesondere aus handelsüblichen Kartuschen, welche manuell, mittels Druckluft oder Batterie betrieben werden, oder aus einem Fass oder Hobbock mittels einer Förderpumpe oder eines Extruders, gegebenenfalls mittels eines Applikationsroboters, auf ein Substrat aufgetragen.
Für eine Anwendung als Beschichtung weist die Zusammensetzung bevorzugt eine bei Raumtemperatur flüssige Konsistenz mit selbstverlaufenden Eigenschaften auf. Gegebenenfalls ist sie leicht thixotrop, so dass die Beschichtung an abschüssigen bis senkrechten Flächen applizierbar ist, ohne sofort wegzufliessen. Sie wird insbesondere appliziert mittels Rolle oder Pinsel oder durch Ausgiessen und Verteilen mittels beispielsweise einem Roller, einem Schaber oder einer Zahntraufel.

Bei der Applikation wird die Zusammensetzung bevorzugt auf mindestens ein Substrat appliziert.
Geeignete Substrate sind insbesondere
- Glas, Glaskeramik, Beton, Mörtel, Backstein, Ziegel, Gips oder Natursteine wie Kalkstein, Granit oder Marmor;
- Metalle und Legierungen, wie Aluminium, Eisen, Stahl oder Buntmetalle, sowie oberflächenveredelte Metalle oder Legierungen, wie verzinkte oder verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe und weitere sogenannte Polymer-Composites;
- Kunststoffe, wie Polyvinylchlorid (Hart- und Weich-PVC), Acrylonitril-Butadien-Styrol-Copolymere (ABS), Polycarbonat (PC), Polyamid (PA), Polyester, Poly(methylmethacrylat) (PMMA), Epoxidharze, Polyurethane (PUR), Polyoxymethylen (POM), Polyolefine (PO), Polyethylen (PE) oder Polypropylen (PP), Ethylen/Propylen-Copolymere (EPM) oder Ethylen/Propylen/Dien-Terpolymere (EPDM), oder faserverstärkte Kunststoffe wie Kohlefaserverstärkte Kunststoffe (CFK), Glasfaser-verstärkte Kunststoffe (GFK) oder Sheet Moulding Compounds (SMC), wobei die Kunststoffe bevorzugt mittels Plasma, Corona oder Flammen oberflächenbehandelt sein können;
- beschichtete Substrate, wie pulverbeschichtete Metalle oder Legierungen;
- Farben oder Lacke, insbesondere Automobildecklacke.
Die Substrate können bei Bedarf vor dem Applizieren der Zusammensetzung vorbehandelt werden, insbesondere durch physikalische und/oder chemische Reinigungsverfahren oder durch das Aufbringen eines Haftvermittlers, einer Haftvermittlerlösung oder eines Primers.
Besonders geeignet ist die Zusammensetzung für den Kontakt mit Substraten, die besonders empfindlich sind auf Störungen durch migrierende Substanzen, insbesondere durch das Ausbilden von Verfärbungen oder Flecken. Dies sind insbesondere feinporige Substrate wie Marmor, Kalkstein oder andere Natursteine, Gips, Zementmörtel oder Beton, aber auch Kunststoffe. Insbesondere auf PVC werden bei der Anwesenheit von Katalysatoren wie beispielsweise DBU oder TMG starke Verfärbungen beobachtet, die sich durch Reinigung nicht entfernen lassen. Solche Effekte werden mit dem Silan der Formel (I) nicht beobachtet.

Verklebt oder abgedichtet werden können zwei gleichartige oder zwei verschiedene Substrate, insbesondere die vorgängig genannten Substrate.

Nach der Aushärtung der Zusammensetzung mit Wasser, insbesondere in Form von Luftfeuchtigkeit, und/oder mit mindestens einem geeigneten Vernetzer wird eine ausgehärtete Zusammensetzung erhalten.

Aus der Anwendung der Zusammensetzung entsteht ein Artikel, welcher mit der Zusammensetzung insbesondere verklebt, abgedichtet oder beschichtet wurde. Beim Artikel handelt es sich insbesondere um ein Bauwerk, insbesondere ein Bauwerk des Hoch- oder Tiefbaus, um ein industriell gefertigtes Gut oder um ein Konsumgut, insbesondere ein Fenster, eine Haushaltmaschine oder ein Transportmittel wie insbesondere ein Automobil, ein Bus, ein Lastkraftwagen, ein Schienenfahrzeug, ein Schiff, ein Flugzeug oder ein Helikopter; oder der Artikel kann ein Anbauteil davon sein.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

Als "Normklima" wird eine Temperatur von 23±1 °C und eine relative Luftfeuchtigkeit von 50±5% bezeichnet.
"EEW" steht für das Epoxid-Equivalentgewicht.

**¹H-NMR-Spektren** wurden auf einem Spektrometer des Typs Bruker Ascend 400 bei 400.14 MHz gemessen; die chemischen Verschiebungen δ sind angegeben in ppm relativ zu Tetramethylsilan (TMS). Kopplungskonstanten J sind angegeben in Hz. Echte und Pseudo-Kopplungsmuster wurden nicht unterschieden.
**Infrarotspektren** (FT-IR) wurden auf einem mit horizontaler ATR-Messeinheit mit Diamant-Kristall ausgestatteten FT-IR Gerät Nicolet iS5 von Thermo Scientific gemessen. Flüssige Proben wurden unverdünnt als Filme aufgetragen, feste Proben wurden in CH₂Cl₂ gelöst. Die Absorptionsbanden sind in Wellenzahlen (cm⁻¹) angegeben (Messfenster: 4000-650 cm⁻¹).
**Gaschromatogramme** (GC) wurden gemessen im Temperaturbereich von 60 bis 320 °C mit einer Aufheizrate von 15 °C/min und 10 min Verweilzeit bei 320 °C. Die Injektortemperatur betrug 250 °C. Es wurde eine Zebron ZB-5 Säule verwendet (L = 30 m, ID = 0.25 mm, dj = 0.5 µm) bei einem Gasfluss von 1.5 ml/Min. Die Detektion erfolgte mittels Flammenionisation (FID), wobei die Signale via Flächenprozent-Methode ausgewertet wurden.
Die **Hautbildungszeit** (HBZ) wurde dadurch bestimmt, dass einige Gramm der Zusammensetzung in einer Schichtdicke von ca. 2 mm auf Pappkarton aufgetragen wurden und im Normklima die Zeitdauer gemessen wurde, bis beim leichten Antippen der Oberfläche der Zusammensetzung mittels einer Pipette aus LDPE erstmals keine Rückstände auf der Pipette mehr zurückblieben.
Die Beschaffenheit der **Oberfläche** wurde haptisch geprüft.
Die mechanischen Eigenschaften **Zugfestigkeit, Bruchdehnung** und **E-Modul** (bei 0-5% und bei 0-50% Dehnung) wurden gemäss DIN EN 53504 bei einer Zuggeschwindigkeit von 200 mm/min. gemessen.
**Viskositäten** wurden auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹) gemessen.

### Herstellung von Hydroxy-Amidinen oder -Guanidinen:

**Guanidin H1:** 1-(2-(2-Hydroxyethoxy)ethyl)-2,3-diisopropylguanidin In einem Rundkolben wurden 23.14 g 2-(2-Aminoethoxy)ethanol (Diglycolamine® Agent, von Huntsman) und 25.24 g N,N'-Diisopropylcarbodiimid (von Sigma-Aldrich) vermischt und die Mischung unter Rühren auf 120 °C erwärmt. In regelmässigen Abständen wurde das Reaktionsgemisch mittels FT-IR-Spektroskopie untersucht. Nach 2 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 56.50 g eines hellgelben, geruchsarmen Öls.
FT-IR: 3354 (O-H), 2963, 2921, 2865, 1616 (C=N), 1524, 1465, 1362, 1337, 1178, 1121, 1066, 884, 829, 715.

**Amidin H2:** 1-(2-Hydroxyethyl)-2-methyl-1,4,5,6-tetrahydropyrimidin In einem Rundkolben wurden 11.91 g N-(3-Aminopropyl)-2-aminoethanol, 15.94 g Trimethylorthoacetat und 0.32 g Lanthan(III)-trifluormethansulfonat unter Rühren während 24 Stunden auf 120 °C erwärmt. Darauf wurde das Reaktionsgemisch im Vakuum von den flüchtigen Bestandteilen befreit und der Rückstand im Vakuum destilliert. Man erhielt 14.44 g eines farblosen geruchsarmen Öls mit einer Siedetemperatur von 130 bis 135 °C bei 0.1 mbar, welches gemäss GC-Spektrum einen Gehalt von 98% Amidin A1 enthielt und beim Stehenlassen bei Raumtemperatur zu einem weissen Feststoff kristallisierte.
¹H-NMR (CDCl₃): δ 1.83 (*quint*., 2 H, J = 5.6, NCH₂C*H₂*CH₂N), 2.02 (*s*, 3 H, CH₃), 3.24 (*t,* 2 H, J = 5.8, NC*H₂*CH₂OH), 3.31 (*m*, 4 H, NC*H₂*CH₂C*H₂*N), 3.69 (*t*, 2 H, J = 5.7, NCH₂C*H₂*OH).
FT-IR: 3214, 3177, 2996, 2925, 2843, 1630, 1542, 1475, 1438, 1380, 1360, 1322, 1294, 1273, 1204, 1191, 1139, 1114, 1095, 1035, 1009, 977, 915, 875, 839, 731.

### verwendete Alkoxysilane:

- AMMO: 3-Aminopropyltrimethoxysilan (Silquest® A-1110, von Momentive)
- VTMO: Vinyltrimethoxysilan (Silquest® A-171, von Momentive)
- VTEO: Vinyltriethoxysilan (Silquest® A-151, von Momentive)
- TEOS: Tetraethoxysilan (=Tetraethylorthosilicat) (Sigma-Aldrich)

### Herstellung eines Silans der Formel (IVa):

### Silan W1: 3-Aminopropyl-dimethoxy-2-(2-aminoethoxy)ethoxysilan

In einem Rundkolben wurden 31.00 g 2-(2-Aminoethoxy)ethanol (Diglycolamine® Agent, von Huntsman) mit 50.25 g AMMO vermischt, während 20 Stunden auf 100 °C erwärmt und bei 100 mbar Methanol abdestilliert. Darauf wurde das Reaktionsgemisch während 2 Stunden am Rotationsverdampfer bei 120 °C und 10 mbar eingeengt. Man erhielt 65.17 g eines farblosen, geruchsarmen Öls.
FT-IR: 3370, 3289, 2928, 2860, 2840, 1663, 1597, 1457, 1411, 1352, 1294, 1243, 1190, 1074, 958, 908, 858, 802, 696.

### Herstellung von Silanen der Formel (I):

### Silan K1: 1-(2-(2-(3-Aminopropyldimethoxysilyloxy)ethoxy)ethyl)-2,3-diisopropylguanidin

In einem Rundkolben wurden 2.31 g des vorgängig hergestellten Guanidins H1 mit 1.79 g AMMO vermischt und unter Stickstoffatmosphäre während 15 Stunden auf 90 °C erwärmt. Darauf wurde das Reaktionsgemisch im Vakuum von den flüchtigen Bestandteilen befreit. Man erhielt 3.79 g eines hellgelben, niedrigviskosen, geruchlosen Öls, welches das Silan K1 in hoher Reinheit enthielt (gemäss NMR-Analyse).
¹H-NMR (CDCl₃): δ 0.66 (*m*, 2 H, CH₂Si), 1.13 (*d*, *J* = 6.4, 12 H, NCH(*CH*₃)₂), 1.55 (*m*, 2 H, C*H*₂CH₂Si), 2.66 (*m*, 2 H, C*H*₂NH₂), 3.27 (*m*, 2 H, N*CH*(CH₃)₂), 3.49-3.66 (*m*, 12 H, SiOCH₃ und CH₂OCH₂CH₂N), 3.89 (*m*, 2 H, CH₂-*CH*₂OSi). FT-IR: 2961, 2929, 2866, 2840, 1633 (C=N), 1516, 1465, 1455, 1361, 1339, 1328, 1183, 1099, 963, 809, 715.

**Silan K2:** 1-(2-(2-(Vinyldimethoxysilyloxy)ethoxy)ethyl)-2,3-diisopropylguanidin In einem Rundkolben wurden 1.25 g des vorgängig hergestellten Guanidins H1 mit 1.51 g VTMO (Vinyltrimethoxysilan) vermischt und unter Stickstoffatmosphäre während 24 Stunden auf 120 °C erwärmt. Darauf wurde das Reaktionsgemisch im Vakuum von den flüchtigen Bestandteilen befreit. Man erhielt 1.54 g eines hellgelben, niedrigviskosen, geruchlosen Öls, das das Silan K2 und nicht umgesetztes Hydroxy-Guanidin H1 in einem Verhältnis von ca. 80:20 enthielt (gemäss NMR-Analyse).
¹H-NMR (CDCl₃) (nur Signale von Silan K2): δ 1.08-1.18 (*s*, 12 H, NCH*Me*₂), 3.27 (*m*, 2 H, NC*H*Me₂), 3.47-3.74 (*m*, 12 H, SiOMe und CH₂OCH₂CH₂N), 3.90 (*m*, 2 H, CH₂OSi), 5.83-6.17 (*m*, 3 H, C*H₂*=C*H*Si).
FT-IR: 3347, 2965, 2927, 2867, 1618 (C=N), 1526, 1465, 1407, 1364, 1338, 1282, 1117, 1066, 1031, 962, 885, 752.

### Silan K3: 1-(2-(2-(Vinyldiethoxysilyloxy)ethoxy)ethyl)-2,3-diisopropylguanidin

In einem Rundkolben wurden 1.81 g des vorgängig hergestellten Guanidins H1 mit 2.98 g VTEO (Vinyltriethoxysilan) vermischt und unter Stickstoffatmosphäre während 48 Stunden auf 120 °C erwärmt. Darauf wurde das Reaktionsgemisch im Vakuum von den flüchtigen Bestandteilen befreit. Man erhielt 2.77 g eines hellgelben, niedrigviskosen, geruchlosen Öls, das das Silan K3 und nicht umgesetztes Hydroxy-Guanidin H1 in einem Verhältnis von ca. 80:20 enthielt (gemäss NMR-Analyse).
¹H-NMR (CDCl₃) (nur Signale von Silan K3): δ 1.08-1.18 (s, 12 H, NCH*Me*₂), 1.30 (*m*, 6 H, C*H₃*CH₂OSi), 3.27 (*m*, 2 H, NC*H*Me₂), 3.55-3.74 (*m*, 6 H, CH₂OCH₂CH₂N), 3.79-3.93 (*m*, 6 H, CH₃C*H*₂OSi), 5.83-6.17 (*m*, 3 H, C*H*₂=C*H*Si).
FT-IR: 3366, 2966, 2927, 2882, 1638 (C=N), 1505, 1455, 1406, 1383, 1362, 1328, 1296, 1075, 1010, 961, 782, 759, 716.

### Silan K4: 1-(2-(2-(Triethoxysilyloxy)ethoxy)ethyl)-2,3-diisopropylguanidin

In einem Rundkolben wurden 1.24 g des vorgängig hergestellten Guanidins H1 mit 1.74 g TEOS (Tetraethoxysilan) vermischt und unter Stickstoffatmosphäre während 24 Stunden auf 120 °C erwärmt. Darauf wurde das Reaktionsgemisch im Vakuum von den flüchtigen Bestandteilen befreit. Man erhielt 2.16 g eines hellgelben, niedrigviskosen, geruchlosen Öls, das das Silan K4 und nicht umgesetztes Hydroxy-Guanidin H1 in einem Verhältnis von ca. 80:20 enthielt (gemäss NMR-Analyse).
¹H-NMR (CDCl₃) (nur Signale von Silan K4): δ 1.08-1.18 (*s*, 12 H, NCH*Me*₂), 1.30 (*m*, 9 H, C*H*₃CH₂OSi), 3.27 (*m*, 2 H, NC*H*Me₂), 3.55-3.74 (*m*, 6 H von CH₂CH₂OCH₂CH₂N), 3.79-3.93 (*m*, 8 H, CH₃C*H*₂OSi und 2 H von CH₂CH₂OCH₂CH₂N).
FT-IR: 3363, 2966, 2928, 2887, 1634 (C=N), 1514, 1465, 1383, 1363, 1338, 1298, 1250, 1073, 967, 792, 712.

### Silan K5: 1-(2-(3-Aminopropyldimethoxysilyloxy)ethyl)-2-methyl-1,4,5,6-tetrahydropyrimidin

In einem Rundkolben wurden 3.59 g des vorgängig hergestellten Amidins H2 mit 4.43 g AMMO vermischt und während 5 Stunden auf 100 °C erwärmt und bei 100 mbar Methanol abdestilliert. Darauf wurde das Reaktionsgemisch während 2 Stunden am Rotationsverdampferbei 120 °C und 10 mbar eingeengt. Man erhielt 6.63 g eines gelben, geruchlosen Öls.
¹H-NMR (CDCl₃): δ 0.67 (*m,* 2 H, CH₂Si), 1.05 (s, 2 H, NH₂), 1.55 (*s*, 2 H, C*H*₂CH₂Si), 1.77-1.85 (*m*, 2 H, N-CH₂-C*H*₂-CH₂-N), 2.0 (s, 3 H, C*H*₃-C), 2.68 (s, 2 H, SiCH₂CH₂C*H*₂NH₂), 3.20-3.37 (m, 6 H, CH₂N), 3.57 (*m,* 6 H, OCH₃), 3.84 (m, 2 H, SiOCH₂).
FT-IR: 3269, 2924, 2838, 1617 (C=N), 1481, 1420, 1376, 1352, 1317, 1292, 1247, 1189, 1079, 1064, 1014, 942, 922, 784, 694.

### Silan K6: 1-(3-(2-(2-(2,3-diisopropylguanidino)ethoxy)ethoxy)dimethoxysilyl)-propyl-2,3-diisopropylguanidin

In einem Rundkolben wurden 8.24 g des vorgängig hergestellten Silans W1 mit 11.13 g N,N'-Diisopropylcarbodiimid vermischt und unter Rühren auf 120 °C erwärmt. In regelmässigen Abständen wurde das Reaktionsgemisch mittels FT-IR-Spektroskopie untersucht. Nach 11 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 15.69 g eines farblosen, geruchsarmen Öls.
¹H-NMR (CDCl₃): δ 0.7 (m, 2 H, CH₂Si), 1.12 (*d,* 24 H, J= 6.1 Hz, *CH₃*-CHN), 1.64 (*s*, 2 H, C*H*₂CH₂Si), 2.99 (*m*, 2 H, SiCH₂CH₂*CH₂*NH), 3.26 (*m*, 2 H, OCH₂*CH₂*NH), 3.48-3.65 (*m*, 12 H, OCH₃ und OCH₂), 3.65-3.8 (*m*, 4 H, CH₃C*H*), 3.89 (*m*, 2 H, SiOCH₂).
FT-IR: 3368, 2961, 2930, 2868, 2840, 1633 (C=N), 1505, 1465, 1360, 1328, 1181, 1081, 964, 811, 712.

### Herstellung von Silangruppen-haltigen Polyethern:

### Polymer STP-1:

Unter Feuchtigkeitsausschluss wurden 1000 g Polyol Acclaim® 12200 (Polyoxypropylendiol mit niedrigem Ungesättigtheitsgrad, von Bayer; OH-Zahl 11.0 mg KOH/g), 43.6 g Isophorondiisocyanat (IPDI; Vestanat® IPDI, von Evonik), 126.4 g Diisodecylphthalat (DIDP) und 0.1 g Bismuttris(neodecanoat) (10 Gew.-% in DIDP) unter stetigem Rühren auf 90 °C aufgeheizt und auf dieser Temperatur belassen, bis der titrimetrisch bestimmte Gehalt an freien Isocyanatgruppen einen stabilen Wert von 0.63 Gew.-% erreicht hatte. Anschliessend wurden 63.0 g N-(3-Trimethoxysilylpropyl)-amino-bernsteinsäurediethylester (Addukt aus 3-Aminopropyltrimethoxysilan und Maleinsäurediethylester; hergestellt nach den Angaben in US 5,364,955) eingemischt und die Mischung bei 90 °C solange gerührt, bis mittels FT-IR-Spektroskopie kein freies Isocyanat mehr nachgewiesen wurde. Der so erhaltene Trimethoxysilangruppen-haltige Polyether mit einem Silan-Equivalentgewicht von ca. 6880 g/Eq (aus den Einsatzmengen berechnet) wurde auf Raumtemperatur abgekühlt und unter Ausschluss von Feuchtigkeit aufbewahrt.

### Polymer STP-2:

Unter Feuchtigkeitsausschluss wurden 1000 g Polyol Acclaim® 12200 (Polyoxypropylendiol mit niedrigem Ungesättigtheitsgrad, von Bayer; OH-Zahl 11.0 mg KOH/g), 43.6 g Isophorondiisocyanat (IPDI; Vestanat® IPDI, von Evonik), 126.4 g Diisodecylphthalat (DIDP) und 0.1 g Bismuttris(neodecanoat) (10 Gew.-% in DIDP) unter stetigem Rühren auf 90 °C aufgeheizt und auf dieser Temperatur belassen, bis der titrimetrisch bestimmte Gehalt an freien Isocyanatgruppen einen stabilen Wert von 0.64 Gew.-% erreicht hatte. Anschliessend wurden 70.6 g N-(3-Triethoxysilylpropyl)-amino-bernsteinsäurediethylester (Addukt aus 3-Aminopropyltriethoxysilan und Maleinsäurediethylester) eingemischt und die Mischung bei 90 °C solange gerührt, bis mittels FT-IR-Spektroskopie kein freies Isocyanat mehr nachgewiesen wurde. Der so erhaltene Triethoxysilangruppen-haltige Polyether mit einem Silan-Equivalentgewicht von ca. 6920 g/Eq (aus den Einsatzmengen berechnet) wurde auf Raumtemperatur abgekühlt und unter Ausschluss von Feuchtigkeit aufbewahrt.

### Verwendete kommerzielle Katalysatoren und Abkürzungen dafür:

- DBU: 1,8-Diazabicyclo[5.4.0]undec-7-en (Lupragen® N 700, von BASF)
- TMG: 1,1,3,3-Tetramethylguanidin (von Sigma-Aldrich)
- IBAY: Bis(ethylacetoacetato)diisobutoxy-titan(IV) (Tyzor® IBAY, von Dorf Ketal)

### Zusammensetzungen auf Basis von Silangruppen-haltigen Polymeren:

Vergleichsbeispiele sind in den Tabellen 1 bis 6 mit "(Ref)" gekennzeichnet.

### Zusammensetzungen Z1 bis Z9:

Eine Zusammensetzung aus 96.5 g Polymer STP-1, 0.5 g Vinyltrimethoxysilan und 3.0 g 3-Aminopropyltrimethoxysilan wurde mit verschiedenen Katalysatoren in der angegebenen Menge gemäss Tabelle 1 vermengt und die Mischung auf Viskosität und Hautbildungszeit (HBZ) im Normklima, vor und nach Lagerung, geprüft. Die Hautbildungszeit dient dabei als Mass für die Aktivität des Katalysators in Bezug auf die Vernetzungsreaktion der Silangruppen, d.h. für die Vernetzungsgeschwindigkeit; die Veränderung der Viskosität und der Hautbildungszeit nach Lagerung sind ein Mass für die Lagerstabilität. Weiterhin wurde die applizierte Mischung nach 24 Stunden im Normklima daraufhin geprüft, ob die Oberfläche wie gewünscht trocken war oder sich ein schmieriger Film gebildet hatte, was ein Anzeichen für das Ausschwitzen des Katalysators aufgrund schlechter Verträglichkeit mit dem ausgehärteten Kunststoff ist, und/oder ob die Oberfläche klebrig war, was ein Anzeichen für eine unvollständige Aushärtung ist. Weiterhin wurde von der Mischung ein Film von 2 mm Dicke hergestellt, während 7 Tagen im Normklima aushärten lassen und auf mechanische Eigenschaften geprüft. Die Ergebnisse sind in Tabelle 1 und 2 wiedergegeben. "Zus." steht für "Zusammensetzung".

**Tabelle 1:**

| **Zus.** | **Katalysator** | **Menge** | **Konzentration¹** | **Viskosität [Pa·s]** | | | **HBZ** | |
|---|---|---|---|---|---|---|---|---|
| | | | | **frisch** | **gelagert²** | **Zunahme** | **frisch** | **gelagert²** |
| **Z1** | Silan K1 | 0.69 g | 1.9 | 21.3 | 28.2 | 32% | 13' | 21' |
| **Z2** | Silan K2 | 0.59 g | 1.9 | 28.8 | 35.5 | 23% | 15' | 12' |
| **Z3** | Silan K3 | 0.63 g | 1.9 | 26.7 | 34.9 | 31% | 16' | 15' |
| **Z4** | Silan K4 | 0.66 g | 1.9 | 27.8 | 36.1 | 30% | 14' | 11' |
| **Z5** | Silan K5 | 0.53 g | 1.9 | 22.4 | 24.7 | 10% | 25' | 24' |
| **Z6** | Silan K6 | 0.46 g | 1.9 | 21.8 | 36.7 | 68% | 13' | 13' |
| **Z7** (Ref) | DBU | 0.28 g | 1.9 | 27.2 | 36.9 | 36% | 25' | 29' |
| **Z8** (Ref) | TMG | 0.21 g | 1.9 | 22.3 | 24.6 | 10% | 65' | 75' |
| **Z9** (Ref) | Amidin H2 | 0.26 g | 1.9 | 22.2 | 25.0 | 13% | 79' | 62' |

| | | | | | | | | |
|---|---|---|---|---|---|---|---|---|
| ¹ mmol Amidin- oder Guanidin-Gruppen auf 100 g Silangruppen-haltiger Polyether. ² während 7 Tagen bei 60 °C in geschlossenem Gebinde. | | | | | | | | |

**Tabelle 2:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **Z1** | trocken | 0.75 MPa | 99% | 0.99 MPa | 0.77 MPa |
| **Z2** | trocken | 0.66 MPa | 84% | 1.26 MPa | 0.81 MPa |
| **Z3** | trocken | 0.72 MPa | 107% | 0.87 MPa | 0.77 MPa |
| **Z4** | trocken | 0.67 MPa | 82% | 1.30 MPa | 0.83 MPa |
| **Z5** | trocken | 0.77 MPa | 118% | 1.21 MPa | 0.80 MPa |
| **Z6** | trocken | 0.73 MPa | 103% | 1.21 MPa | 0.82 MPa |
| **Z7** (Ref) | schmierig | 0.58 MPa | 72% | 1.16 MPa | 0.77 MPa |
| **Z8** (Ref) | klebrig | 0.62 MPa | 90% | 1.19 MPa | 0.75 MPa |
| **Z9** (Ref) | trocken | 0.67 MPa | 90% | 1.19 MPa | 0.80 MPa |

### Zusammensetzungen Z10 bis Z14:

Eine Zusammensetzung aus 95.9 g Polymer STP-2, 0.4 g Vinyltriethoxysilan und 3.7 g N-(2-Aminoethyl)-3-aminopropyltriethoxysilan wurde mit verschiedenen Katalysatoren in der angegebenen Menge gemäss Tabelle 3 vermengt und die Mischung wie für Zusammensetzung Z1 beschrieben auf Viskosität, Hautbildungszeit (HBZ), Oberflächenbeschaffenheit und mechanische Eigenschaften geprüft. Die Ergebnisse sind in Tabelle 3 und 4 wiedergegeben. "Zus." steht für "Zusammensetzung".

**Tabelle 3:**

| **Zus.** | **Katalysator** | **Menge** | **Konzentration**¹ | **Viskosität [Pa·s]** | | | **HBZ** | |
|---|---|---|---|---|---|---|---|---|
| | | | | **frisch** | **gelagert²** | **Zunahme** | **frisch** | **gelagert**² |
| **Z10** | Silan K1 | 1.38 g | 3.8 | 28.1 | 33.2 | 18% | 1h 47' | 1h 15' |
| **Z11** | Silan K3 | 1.25 g | 3.8 | 28.9 | 34.4 | 19% | 1h 20' | 1h 8' |
| **Z12** | Silan K4 | 1.31 g | 3.8 | 28.9 | 34.6 | 20% | 2h 25' | 1h 10' |
| **Z13** (Ref) | DBU | 0.55 g | 3.8 | 48.8 | 58.1 | 19% | 2h 7' | 2h 35' |
| **Z14** (Ref) | TMG | 0.42 g | 3.8 | 44.5 | 53.4 | 20% | >12h | >12h |

| | | | | | | | | |
|---|---|---|---|---|---|---|---|---|
| ¹ mmol Amidin- oder Guanidin-Gruppen auf 100 g Silangruppen-haltiger Polyether. ² während 7 Tagen bei 60 °C in geschlossenem Gebinde. | | | | | | | | |

**Tabelle 4:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **Z10** | leicht klebrig | 0.65 MPa | 139% | 0.73 MPa | 0.56 MPa |
| **Z11** | leicht klebrig | 0.65 MPa | 139% | 0.51 MPa | 0.55 MPa |
| **Z12** | leicht klebrig | 0.62 MPa | 123% | 0.63 MPa | 0.58 MPa |
| **Z13** (Ref) | schmierig, stark klebrig | 0.43 MPa | 157% | 0.28 MPa | 0.28 MPa |
| **Z14** (Ref) | sehr stark klebrig | n.b. | n.b. | n.b. | n.b. |

| | | | | | |
|---|---|---|---|---|---|
| n.b. = nicht bestimmt bzw. nicht messbar. | | | | | |

### Zusammensetzungen Z15 bis Z18:

In einem Planetenmischer wurden 36.2 g Polymer STP-1, 60.2 g gemahlene Kreide (Omyacarb® 5 GU, von Omya), 1.2 g Thixotropierpaste hergestellt wie nachstehend beschrieben, 1.2 g Vinyltrimethoxysilan, 1.2 g 3-Aminopropyltrimethoxysilan sowie verschiedene Katalysatoren in der angegebenen Menge gemäss Tabelle 6 vermengt und die Mischung wie für Zusammensetzung Z1 beschrieben auf Hautbildungszeit (HBZ), Oberflächenbeschaffenheit und mechanische Eigenschaften geprüft. Die Ergebnisse sind in Tabelle 5 wiedergegeben. "Zus." steht für "Zusammensetzung".
Die Thixotropierpaste wurde hergestellt, indem in einem Vakuummischer 300 g Diisodecylphthalat (Palatinol® Z, von BASF) und 48 g 4,4'-Methylendiphenyldiisocyanat (Desmodur® 44 MC L, von Bayer) vorgelegt und leicht aufgewärmt und anschliessend unter starkem Rühren 27 g n-Butylamin langsam zugetropft wurden. Die entstehende Paste wurde unter Vakuum und Kühlung eine Stunde weitergerührt.

**Tabelle 5:**

| **Zus.** | **Katalysator** | **M enge** | **Konzentration**¹ | **HBZ** | **Oberfläche nach 24h** | **Zugfestigkeit** | **Bruchdehnung** | **E-Modul [MPa]** | |
|---|---|---|---|---|---|---|---|---|---|
| | | | | | | | | **0-5%** | **0-100%** |
| **Z15** | Silan K1 | 0.15 g | 0.4 | 25' | trocken | 3.0 MPa | 106% | 5.9 | 2.8 |
| **Z16** | Silan K3 | 0.27 g | 0.8 | 11' | trocken | 2.9 MPa | 116% | 5.3 | 2.6 |
| **Z17** | Silan K4 | 0.29 g | 0.8 | 17' | trocken | 3.1 MPa | 103% | 5.3 | 3.0 |
| **Z18** (Ref) | DBU | 0.12 g | 0.8 | 25' | leicht schmierig | 2.5 MPa | 103% | 6.1 | 2.8 |

| | | | | | | | | | |
|---|---|---|---|---|---|---|---|---|---|
| ¹ mmol Amidin- oder Guanidin-Gruppen auf 100 g Zusammensetzung. | | | | | | | | | |

### Zusammensetzungen Z19 bis Z22:

In einem Planetenmischer wurden 36.2 g Polymer STP-2, 60.2 g gemahlene Kreide (Omyacarb® 5 GU, von Omya), 1.2 g Thixotropierpaste, hergestellt wie für Zusammensetzung Z27 beschrieben, 1.2 g Vinyltriethoxysilan, 1.2 g 3-Aminopropyltriethoxysilan sowie verschiedene Katalysatoren in der angegebenen Menge gemäss Tabelle 7 vermengt und die Mischung wie für Zusammensetzung Z1 beschrieben auf Hautbildungszeit (HBZ), Oberflächenbeschaffenheit und mechanische Eigenschaften geprüft. Die Ergebnisse sind in Tabelle 6 wiedergegeben. "Zus." steht für "Zusammensetzung".

**Tabelle 6:**

| **Zus.** | **Katalysator** | **Menge** | **Konzentration**¹ | **HBZ** | **Oberfläche nach 24h** | **Zugfestigkeit** | **Bruchdehnung** | **E-Modul [MPa] 0-5% 0-100%** | |
|---|---|---|---|---|---|---|---|---|---|
| **Z19** | Silan K1 | 0.98 g | 2.6 | 118' | leicht klebrig | 2.6 MPa | 137% | 5.6 | 2.3 |
| **Z19** | Silan K3 | 0.89 g | 2.6 | 85' | trocken | 3.1 MPa | 181% | 5.2 | 2.4 |
| **Z20** | Silan K4 | 0.93 g | 2.6 | 100' | fast trocken | 4.3 MPa | 156% | 5.2 | 3.1 |
| **Z22** (Ref) | DBU | 0.40 g | 2.6 | 83' | leicht schmierig | 2.5 MPa | 155% | 4.0 | 2.0 |

| | | | | | | | | | |
|---|---|---|---|---|---|---|---|---|---|
| mmol Amidin- oder Guanidin-Gruppen auf 100 g Silangruppen-haltiger Polyether. | | | | | | | | | |

## Patentansprüche

1. Silan der Formel (I), 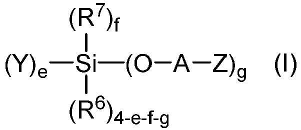 wobei
Z für eine über ein Stickstoffatom gebundene Amidin- oder GuanidinGruppe steht,
A für einen zweiwertigen aliphatischen oder cycloaliphatischen oder arylaliphatischen Kohlenwasserstoff-Rest mit 2 bis 20 C-Atomen, welcher gegebenenfalls Ether-Sauerstoff oder sekundären oder tertiären Amin-Stickstoff enthält, steht,
e für 0 oder 1 steht, f für 0 oder 1 oder 2 steht und g für eine ganze Zahl von 1 bis 4 steht, wobei die Summe (e+f+g) für eine ganze Zahl von 1 bis 4 steht,
R⁶ entweder für einen Alkoxy-Rest mit 1 bis 12 C-Atomen, welcher gegebenenfalls Ether-Sauerstoff enthält, oder für einen Ketoximato-Rest mit 1 bis 13 C-Atomen steht,
R⁷ für einen einwertigen Kohlenwasserstoff-Rest mit 1 bis 12 C-Atomen steht, und
Y für einen einwertigen Kohlenwasserstoff-Rest mit 1 bis 20 C-Atomen, welcher gegebenenfalls eine endständige Mercaptogruppe, Epoxidgruppe, (Meth)acrylgruppe, Amidingruppe, Guanidingruppe, Urethangruppe oder Harnstoffgruppe oder eine endständige Aminogruppe der Formel -NHR⁸ aufweist und welcher gegebenenfalls Ether-Sauerstoff oder sekundären Amin-Stickstoff enthält, steht, wobei R⁸ für einen Wasserstoff-Rest oder einen Alkyl- oder Cycloalkyl- oder Aralkyl-Rest mit 1 bis 8 C-Atomen oder für einen Rest 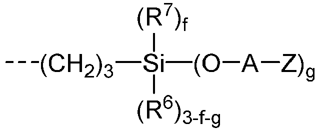 steht, wobei das Silan der Formel (I) kein Stickstoffatom enthält, welches direkt an einen aromatischen Ring gebunden oder Teil eines heteroaromatischen Ringsystems ist.

2. Silan gemäss Anspruch 1, **dadurch gekennzeichnet, dass**
Z für 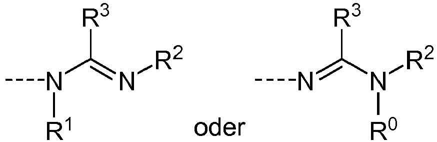 steht, wobei
R⁰ für einen Wasserstoff-Rest oder für einen Alkyl- oder Cycloalkyl- oder Aralkyl-Rest mit 1 bis 8 C-Atomen steht,
R¹ für einen Wasserstoff-Rest oder für einen Alkyl- oder Cycloalkyl- oder Aralkyl-Rest mit 1 bis 8 C-Atomen steht oder zusammen mit R² für R⁹ steht,
R² für einen Wasserstoff-Rest oder für einen Alkyl-, Cycloalkyl- oder Aralkyl-Rest mit 1 bis 18 C-Atomen, welcher gegebenenfalls Ether-Sauerstoff oder tertiären Amin-Stickstoff enthält, oder zusammen mit R¹ für R⁹ steht, R³ für -NR⁴R⁵ oder für einen Wasserstoff-Rest oder für einen Alkyl- oder Cycloalkyl- oder Aralkyl-Rest mit 1 bis 12 C-Atomen steht,
R⁴ und R⁵ unabhängig voneinander jeweils für einen Wasserstoff-Rest oder für einen Alkyl-, Cycloalkyl- oder Aralkyl-Rest mit 1 bis 18 C-Atomen, welcher gegebenenfalls Ether-Sauerstoff oder tertiären Amin-Stickstoff enthält, stehen, und
R⁹ für 1,2-Ethylen oder 1,2-Propylen oder 1,3-Propylen oder 1,3-Butylen oder 1,3-Pentylen steht,
wobei
R² und R⁰ auch zusammen für einen Alkylen-Rest mit 3 bis 6 C-Atomen, welcher gegebenenfalls Ether-Sauerstoff oder tertiären Amin-Stickstoff enthält, stehen können,
R² und R³ auch zusammen für einen Alkylen-Rest mit 3 bis 6 C-Atomen stehen können,
R⁴ und R⁵ auch zusammen für einen Alkylen-Rest mit 4 bis 7 C-Atomen, welcher gegebenenfalls Ether-Sauerstoff oder tertiären Amin-Stickstoff enthält, stehen können, und
R² und R⁵ auch zusammen für einen Alkylen-Rest mit 2 bis 12 C-Atomen stehen können.

3. Silan gemäss einem der Ansprüche 1 oder 2, **dadurch gekennzeichnet, dass** A ausgewählt ist aus der Gruppe bestehend aus 1,2-Ethylen, 1,2-Propylen, 1,3-Propylen, 1,4-Butylen, 1,1-Dimethyl-1,2-ethylen, 1,5-Pentylen, 1,6-Hexylen, (1,5,5-Trimethylcyclohexan-1-yl)methan-1,3, 3-Oxa-1,5-pentylen und 3,6-Dioxa-1,8-octylen.

4. Silan gemäss einem der Ansprüche 1 bis 3, **dadurch gekennzeichnet, dass** Y ausgewählt ist aus der Gruppe bestehend aus Methyl, Octyl, Isooctyl, Phenyl, Vinyl, 3-Aminopropyl, N-(2-Aminoethyl)-3-aminopropyl, 3-Mercaptopropyl, 3-Glycidoxypropyl, 3-Acryloxypropyl, 3-Methacryloxypropyl und einem Rest der Formel

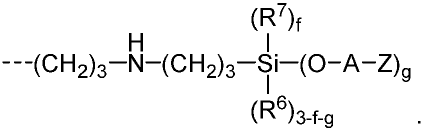

5. Silan gemäss einem der Ansprüche 2 bis 4, **dadurch gekennzeichnet, dass** Z für 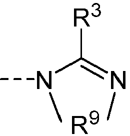 steht, wobei R³ für einen Wasserstoff-Rest oder für einen Alkyl-, Cycloalkyl- oder Aralkyl-Rest mit 1 bis 12 C-Atomen steht.

6. Silan gemäss einem der Ansprüche 2 bis 4, **dadurch gekennzeichnet, dass** Z für 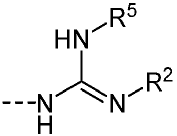 steht, wobei R² und R⁵ unabhängig voneinander jeweils für einen Alkyl-, Cycloalkyl- oder Aralkyl-Rest mit 1 bis 12 C-Atomen, welcher gegebenenfalls Ether-Sauerstoff oder tertiären Amin-Stickstoff enthält, stehen.

7. Verfahren zur Herstellung des Silans gemäss einem der Ansprüche 1 bis 6, **dadurch gekennzeichnet, dass**,
- mindestens ein Amin der Formel (IIa) oder (IIb),
HO-A-NHR¹ (IIa)
HO-A-NH-R⁹-NH₂ (IIb)
- gegebenenfalls mindestens ein Amin der Formel R²-NH-R⁰,
- mindestens ein Reagens zur Einführung von Amidin- oder Guanidin-Gruppen und
- mindestens ein Alkoxy- oder Ketoximato-Silan miteinander umgesetzt werden.

8. Amidin- oder Guanidingruppen-haltiges Umsetzungsprodukt, welches aus einem Silan gemäss einem der Ansprüche 1 bis 6, bei welchem (e+f+g) für 1 oder 2 oder 3 steht, durch Kondensation mit mindestens einer Silanolgruppen-haltigen Verbindung erhalten wird.

9. Umsetzungsprodukt gemäss Anspruch 8, **dadurch gekennzeichnet, dass** es durch Hydrolyse- und nachfolgende Kondensationsreaktionen allein aus dem Silan gemäss einem der Ansprüche 1 bis 6, bei welchem (e+f+g) für 1 oder 2 oder 3 steht, erhalten wird und ein Silanol- und/oder Siloxangruppen aufweisendes oligomeres Folgeprodukt des Silans darstellt.

10. Umsetzungsprodukt gemäss Anspruch 8, **dadurch gekennzeichnet, dass** es aus der Kondensation mit mindestens einem Siliconöl der Formel 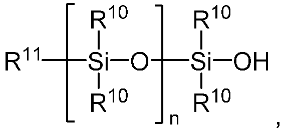 wobei
n für eine ganze Zahl im Bereich von 3 bis 200 steht,
R¹⁰ für einen einwertigen Kohlenwasserstoffrest mit 1 bis 6 C-Atomen steht, und
R¹¹ für einen Hydroxyl-Rest oder für einen Alkyl- oder Alkoxy- oder Ketoximato-Rest mit 1 bis 13 C-Atomen steht,
erhalten wird.

11. Verwendung des Silans gemäss einem der Ansprüche 1 bis 6 und/oder eines Umsetzungsproduktes davon gemäss einem der Ansprüche 8 bis 10 als Katalysator in härtbaren Zusammensetzungen.

12. Verwendung gemäss Anspruch 11, **dadurch gekennzeichnet, dass** die härtbare Zusammensetzung mindestens ein Silangruppen-haltiges Polymer enthält.

13. Verwendung gemäss Anspruch 12, **dadurch gekennzeichnet, dass** das Silangruppen-haltige Polymer ausgewählt ist aus der Gruppe bestehend aus Polyorganosiloxanen mit endständigen Silangruppen und Silangruppen-haltigen organischen Polymeren.

14. Zusammensetzung umfassend mindestens ein Silan gemäss einem der Ansprüche 1 bis 6 und/oder mindestens ein Umsetzungsprodukt gemäss einem der Ansprüche 8 bis 10 und mindestens ein Silangruppen-haltiges Polymer.

15. Zusammensetzung gemäss Anspruch 14, **dadurch gekennzeichnet, dass** sie einen Klebstoff oder einen Dichtstoff oder eine Beschichtung darstellt.

## Claims

1. A silane of the formula (I), in which 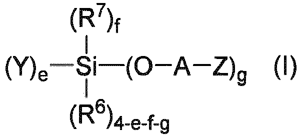
Z is an amidine or guanidine group bonded via a nitrogen atom,
A is a divalent aliphatic or cycloaliphatic or arylaliphatic hydrocarbon radical having 2 to 20 C atoms, which optionally comprises ether-oxygen or secondary or tertiary amine-nitrogen,
e is 0 or 1, f is 0 or 1 or 2, and g is an integer from 1 to 4, and the sum (e+f+g) is an integer from 1 to 4,
R⁶ either is an alkoxy radical having 1 to 12 C atoms which optionally contains ether-oxygen, or is a ketoximato radical having 1 to 13 C atoms,
R⁷ is a monovalent hydrocarbon radical having 1 to 12 C atoms, and
Y is a monovalent hydrocarbon radical having 1 to 20 C atoms which optionally has a terminal mercapto group, epoxide group, (meth)acryloyl group, amidine group, guanidine group, urethane group or urea group or has a terminal amino group of the formula -NHR⁸, and which optionally contains ether-oxygen or secondary amine-nitrogen, where R⁸ is a hydrogen radical or an alkyl or cycloalkyl or aralkyl radical having 1 to 8 C atoms or is a radical 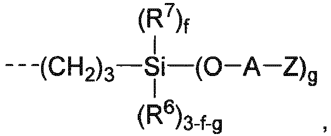
where the silane of the formula (I) contains no nitrogen atom which is bonded directly to an aromatic ring or is part of a heteroaromatic ring system.

2. The silane as claimed in claim 1, **characterized in that** Z is 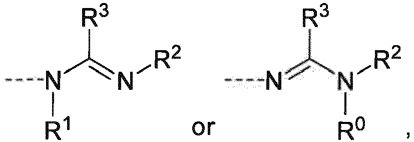 where
R⁰ is a hydrogen radical or is an alkyl or cycloalkyl or aralkyl radical having 1 to 8 C atoms,
R¹ is a hydrogen radical or is an alkyl or cycloalkyl or aralkyl radical having 1 to 8 C atoms, or together with R² is R⁹,
R² is a hydrogen radical or is an alkyl, cycloalkyl or aralkyl radical having 1 to 18 C atoms, which optionally contains ether-oxygen or tertiary amine-nitrogen, or together with R¹ is R⁹,
R³ is -NR⁴R⁵ or is a hydrogen radical or is an alkyl or cycloalkyl or aralkyl radical having 1 to 12 C atoms,
R⁴ and R⁵ independently of one another are each a hydrogen radical or are an alkyl, cycloalkyl or aralkyl radical having 1 to 18 C atoms, which optionally contains ether-oxygen or tertiary amine-nitrogen, and
R⁹ is 1,2-ethylene or 1,2-propylene or 1,3-propylene or 1,3-butylene or 1,3-pentylene,
where
R² and R⁰ may also together be an alkylene radical having 3 to 6 C atoms, which optionally contains ether-oxygen or tertiary amine-nitrogen, R² and R³ may also together be an alkylene radical having 3 to 6 C atoms,
R⁴ and R⁵ may also together be an alkylene radical having 4 to 7 C atoms, which optionally contains ether-oxygen or tertiary amine-nitrogen, and
R² and R⁵ may also together be an alkylene radical having 2 to 12 C atoms.

3. The silane as claimed in either of claims 1 and 2, **characterized in that** A is selected from the group consisting of 1,2-ethylene, 1,2-propylene, 1,3-propylene, 1,4-butylene, 1,1-dimethyl-1,2-ethylene, 1,5-pentylene, 1,6-hexylene, (1,5,5-trimethylcyclohexan-1-yl)methane-1,3, 3-oxa-1,5-pentylene and 3,6-dioxa-1,8-octylene.

4. The silane as claimed in any of claims 1 to 3, **characterized in that** Y is selected from the group consisting of methyl, octyl, isooctyl, phenyl, vinyl, 3-aminopropyl, N-(2-aminoethyl)-3-aminopropyl, 3-mercaptopropyl, 3-glycidyloxypropyl, 3-acryloyloxypropyl, 3-methacryloyloxypropyl, and a radical of the formula

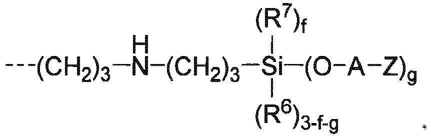

5. The silane as claimed in any of claims 2 to 4, **characterized in that** Z is 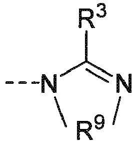 where R³ is a hydrogen radical or is an alkyl, cycloalkyl or aralkyl radical having 1 to 12 C atoms.

6. The silane as claimed in any of claims 2 to 4, **characterized in that** Z is 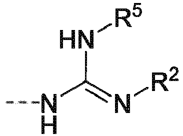 where R² and R⁵ independently of one another are each an alkyl, cycloalkyl or aralkyl radical having 1 to 12 C atoms, which optionally contains ether-oxygen or tertiary amine-nitrogen.

7. A process for preparing the silane as claimed in any of claims 1 to 6, **characterized in that**
- at least one amine of the formula (IIa) or (IIb),
HO-A-NHR¹ (IIa)
HO-A-NH-R⁹-NH₂ (IIb)
- optionally at least one amine of the formula R²-NH-R⁰,
- at least one reagent for introducing amidine or guanidine groups, and
- at least one alkoxy- or ketoximato-silane are reacted with one another.

8. A reaction product containing amidine or guanidine groups which is obtained from a silane as claimed in any of claims 1 to 6, wherein (e+f+g) is 1 or 2 or 3, by condensation with at least one compound containing silanol groups.

9. The reaction product as claimed in claim 8, **characterized in that** it is obtained by hydrolysis and subsequent condensation reactions solely from the silane as claimed in any of claims 1 to 6 wherein (e+f+g) is 1 or 2 or 3, and represents an oligomeric descendant of the silane, containing silanol and/or siloxane groups.

10. The reaction product as claimed in claim 8, **characterized in that** it is obtained from the condensation with at least one silicone oil of the formula 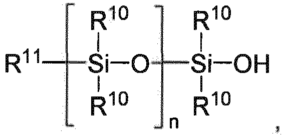 where
n is an integer in the range from 3 to 200,
R¹⁰ is a monovalent hydrocarbon radical having 1 to 6 C atoms, and
R¹¹ is a hydroxyl radical or is an alkyl or alkoxy or ketoximato radical having 1 to 13 C atoms.

11. The use of the silane as claimed in any of claims 1 to 6 and/or of a reaction product thereof as claimed in any of claims 8 to 10 as a catalyst in curable compositions.

12. The use as claimed in claim 11, **characterized in that** the curable composition comprises at least one polymer containing silane groups.

13. The use as claimed in claim 12, **characterized in that** the polymer containing silane groups is selected from the group consisting of polyorganosiloxanes having terminal silane groups and organic polymers containing silane groups.

14. A composition comprising at least one silane as claimed in any of claims 1 to 6 and/or at least one reaction product as claimed in any of claims 8 to 10 and at least one polymer containing silane groups.

15. The composition as claimed in claim 14, **characterized in that** it constitutes an adhesive or a sealant or a coating.

## Revendications

1. Silane de formule (I) 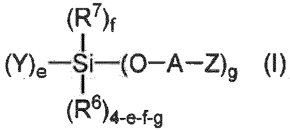 dans laquelle
Z représente un groupe amidine ou guanidine relié par un atome d'azote,
A représente un radical hydrocarboné bivalent aliphatique ou cycloaliphatique ou aryl-aliphatique de 2 à 20 atomes C, qui contient éventuellement un oxygène d'éther ou un azote d'amine secondaire ou tertiaire,
e représente 0 ou 1, f représente 0 ou 1 ou 2, et g représente un nombre entier de 1 à 4, la somme (e+f+g) représentant un nombre entier de 1 à 4,
R⁶ représente un radical alcoxy de 1 à 12 atomes C, qui contient éventuellement un oxygène d'éther, ou un radical cétoximato de 1 à 13 atomes C,
R⁷ représente un radical hydrocarboné monovalent de 1 à 12 atomes C, et
Y représente un radical hydrocarboné monovalent de 1 à 20 atomes C, qui comprend éventuellement un groupe mercapo terminal, un groupe époxyde, un groupe (méth)acrylique, un groupe amidine, un groupe guanidine, un groupe uréthane ou un groupe urée ou un groupe amino terminal de formule -NHR⁸, et qui contient éventuellement un oxygène d'éther ou un azote d'amine secondaire, R⁸ représentant un radical hydrogène ou un radical alkyle ou cycloalkyle ou aralkyle de 1 à 8 atomes C ou un radical 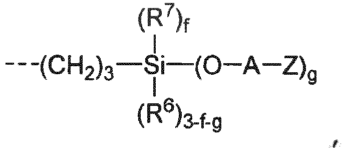
le silane de formule (I) ne contenant pas d'atome d'azote qui est relié directement à un cycle aromatique ou qui fait partie d'un système cyclique hétéroaromatique.

2. Silane selon la revendication 1, **caractérisé en ce que** Z représente 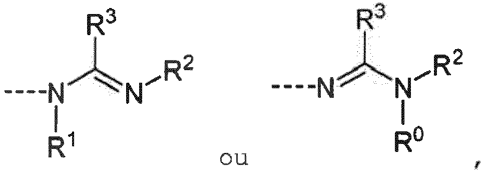
R⁰ représentant un radical hydrogène ou un radical alkyle ou cycloalkyle ou aralkyle de 1 à 8 atomes C,
R¹ représentant un radical hydrogène ou un radical alkyle ou cycloalkyle ou aralkyle de 1 à 8 atomes C, ou représentant R⁹ conjointement avec R²,
R² représentant un radical hydrogène ou un radical alkyle, cycloalkyle ou aralkyle de 1 à 18 atomes C, qui contient éventuellement un oxygène d'éther ou un azote d'amine tertiaire, ou représentant R⁹ conjointement avec R¹,
R³ représentant -NR⁴R⁵ ou un radical hydrogène ou un radical alkyle ou cycloalkyle ou aralkyle de 1 à 12 atomes C,
R⁴ et R⁵ représentant chacun indépendamment l'un de l'autre un radical hydrogène ou un radical alkyle, cycloalkyle ou aralkyle de 1 à 18 atomes C, qui contient éventuellement un oxygène d'éther ou un azote d'amine tertiaire, et
R⁹ représentant 1,2-éthylène ou 1,2-propylène ou 1,3-propylène ou 1,3-butylène ou 1,3-pentylène,
R² et R⁰ pouvant également représenter ensemble un radical alkylène de 3 à 6 atomes C, qui contient éventuellement un oxygène d'éther ou un azote d'amine tertiaire,
R² et R³ pouvant également représenter ensemble un radical alkylène de 3 à 6 atomes C,
R⁴ et R⁵ pouvant également représenter ensemble un radical alkylène de 4 à 7 atomes C, qui contient éventuellement un oxygène d'éther ou un azote d'amine tertiaire, et
R² et R⁵ peuvent également représenter ensemble un radical alkylène de 2 à 12 atomes C.

3. Silane selon l'une quelconque des revendications 1 ou 2, **caractérisé en ce qu'**A est choisi dans le groupe constitué par 1,2-éthylène, 1,2-propylène, 1,3-propylène, 1,4-butylène, 1,1-diméthyl-1,2-éthylène, 1,5-pentylène, 1,6-hexylène, (1,5,5-triméthylcyclohexan-1-yl)méthane-1,3, 3-oxa-1,5-pentylène et 3,6-dioxa-1,8-octylène.

4. Silane selon l'une quelconque des revendications 1 à 3, **caractérisé en ce qu'**Y est choisi dans le groupe constitué par méthyle, octyle, isooctyle, phényle, vinyle, 3-aminopropyle, N-(2-aminoéthyl)-3-aminopropyle, 3-mercaptopropyle, 3-glycidoxypropyle, 3-acryloxypropyle, 3-méthacryloxypropyle et un radical de formule

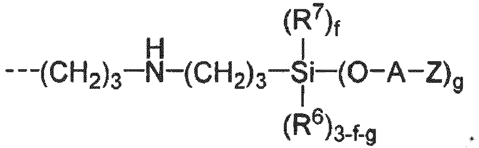

5. Silane selon l'une quelconque des revendications 2 à 4, **caractérisé en ce que** Z représente 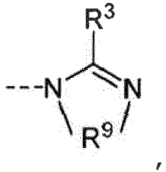 R3 représentant un radical hydrogène ou un radical alkyle, cycloalkyle ou aralkyle de 1 à 12 atomes C.

6. Silane selon l'une quelconque des revendications 2 à 4, **caractérisé en ce que** Z représente 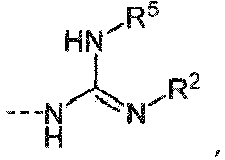 R² et R⁵ représentant chacun indépendamment l'un de l'autre un radical alkyle, cycloalkyle ou aralkyle de 1 à 12 atomes C, qui contient éventuellement un oxygène d'éther ou un azote d'amine tertiaire.

7. Procédé de fabrication du silane selon l'une quelconque des revendications 1 à 6, **caractérisé en ce que**
- au moins une amine de formule (IIa) ou (IIb)
HO-A-NHR¹ (IIa)
HO-A-NH-R⁹-NH₂ (IIb)
- éventuellement au moins une amine de formule R²-NH-R⁰,
- au moins un réactif pour l'introduction de groupes amidine ou guanidine, et
- au moins un alcoxy- ou cétoximato-silane sont mis en réaction les uns avec les autres.

8. Produit de réaction contenant des groupes amidine ou guanidine, qui est obtenu à partir d'un silane selon l'une quelconque des revendications 1 à 6, dans lequel (e+f+g) représente 1 ou 2 ou 3, par condensation avec au moins un composé contenant des groupes silanol.

9. Produit de réaction selon la revendication 8, **caractérisé en ce qu'**il est obtenu par des réactions d'hydrolyse, puis de condensation uniquement à partir du silane selon l'une quelconque des revendications 1 à 6, dans lequel (e+f+g) représente 1 ou 2 ou 3, et constitue un produit oligomère dérivé du silane comprenant des groupes silanol et/ou siloxane.

10. Produit de réaction selon la revendication 8, **caractérisé en ce qu'**il est obtenu par condensation avec au moins une huile de silicone de formule 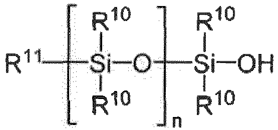 dans laquelle
n représente un nombre entier dans la plage allant de 3 à 200,
R¹⁰ représente un radical hydrocarboné monovalent de 1 à 6 atomes C, et
R¹¹ représente un radical hydroxyle ou un radical alkyle ou alcoxy ou cétoximato de 1 à 13 atomes C.

11. Utilisation du silane selon l'une quelconque des revendications 1 à 6 et/ou d'un produit de réaction de celui-ci selon l'une quelconque des revendications 8 à 10 en tant que catalyseur dans des compositions durcissables.

12. Utilisation selon la revendication 11, **caractérisée en ce que** la composition durcissable contient au moins un polymère contenant des groupes silane.

13. Utilisation selon la revendication 12, **caractérisée en ce que** le polymère contenant des groupes silane est choisi dans le groupe constitué par les polyorganosiloxanes contenant des groupes silane terminaux et les polymères organiques contenant des groupes silane.

14. Composition comprenant au moins un silane selon l'une quelconque des revendications 1 à 6 et/ou au moins un produit de réaction selon l'une quelconque des revendications 8 à 10 et au moins un polymère contenant des groupes silane.

15. Composition selon la revendication 14, **caractérisée en ce qu'**il s'agit d'un adhésif ou d'un agent d'étanchéité ou d'un revêtement.
